(19) Europäisches Patentamt
European Patent Office
Office européen des brevets

(11) EP 4 268 808 A1

(12) **EUROPEAN PATENT APPLICATION**
published in accordance with Art. 153(4) EPC

(43) Date of publication:
**01.11.2023 Bulletin 2023/44**

(21) Application number: **21911593.8**

(22) Date of filing: **23.12.2021**

(51) International Patent Classification (IPC):
***A61K 9/51*** *(2006.01)* ***A61K 48/00*** *(2003.01)*
***A61K 47/26*** *(2006.01)* ***A61K 47/10*** *(2017.01)*
***A61P 1/16*** *(2006.01)* ***A61P 31/20*** *(2006.01)*

(52) Cooperative Patent Classification (CPC):
**A61K 9/51; A61K 47/10; A61K 47/26; A61K 48/00; A61P 1/16; A61P 31/20**

(86) International application number:
**PCT/KR2021/019776**

(87) International publication number:
**WO 2022/139528 (30.06.2022 Gazette 2022/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.12.2020 KR 20200183885**

(71) Applicant: **EnhancedBio Inc.**
**Seoul 04779 (KR)**

(72) Inventors:
- **LEE, Hyukjin**
  **Seoul 06557 (KR)**
- **JEONG, Michaela**
  **Seoul 02531 (KR)**
- **KIM, Minjeong**
  **Seoul 06544 (KR)**

(74) Representative: **Bates, Philip Ian**
**Reddie & Grose LLP**
**The White Chapel Building**
**10 Whitechapel High Street**
**London E1 8QS (GB)**

Remarks:
The complete document including Reference Table(s) and the Sequence Listing(s) can be downloaded from the EPO website

(54) **LIPID NANOPARTICLES COMPRISING MANNOSE OR USES THEREOF**

(57) The present invention relates to lipid nanoparticles comprising mannose or a use thereof. The lipid nanoparticles according to an embodiment are specific for liver tissues and/or LSEC, have excellent biological affinity, and can deliver gene therapeutic agents, etc. at high efficiency, thus finding advantageous applications in the relevant technical fields such as lipid nanoparticle-mediated gene therapy, etc.

【FIG. 11】

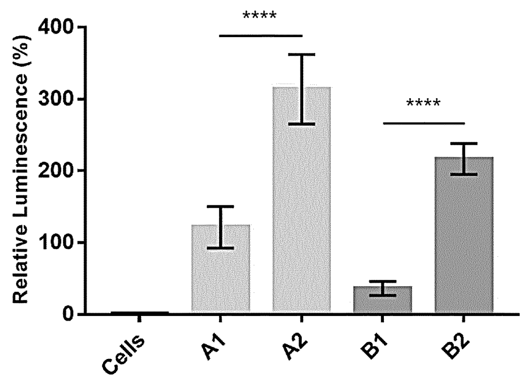


EP 4 268 808 A1

## Description

[TECHNICAL FIELD]

The present invention relates to lipid nanoparticles comprising mannose or uses thereof.

[BACKGROUND ART]

In the pharmaceutical formulation industry, the drug delivery system (DDS), designed to efficiently deliver the required amount of the drug by reducing side effects of the drug and maximizing efficacy and effects, is a high value-added core technology which can create economic benefits comparable to that of new drug development and has great potential for success and its purpose is to improve the quality of patient treatment by making drug administration more efficient. The solubilization technology of poorly soluble drugs belonging to the drug absorption promotion technology, which is one of the core technologies of the drug delivery system, is considered the most reasonable way to reduce the development cost of new drug substances and at the same time increase the added value of currently marketed drugs. In particular, the development of improved new drugs through the development of drug solubilization technology in a situation where new drug development conditions are poor as in Korea is a field that can create enormous added value at a low cost.

Gene therapy using a genetic drug delivery system is established in a large hope of modifying genetic binding and to treat numerous diseases. In the successful and securely performing this gene therapy, the effective gene delivery is one of the main challenges and the virus delivery system was proved to be effective in gene delivery. However, due to some defects such as immunogenicity, limitation of the inserted DNA size and difficulties of mass production, the use of viruses are limited as a gene delivery system. Non-viral gene carriers such as cationic liposome and polymers began to be noted as an alternative means of a viral system.

Improved stability profile and ease of manufacturing and operation of the polymer delivery system triggered studies on the design and synthesis of a non-toxic and biodegradable polymer carrier for effective and safe gene delivery. Poly(L-lysine), polyethylenimine, starburst, polyamidoamine dendrimer, and cationic liposome voluntarily, and the like can be self-assembled and compress plasmid DNA (pDNA) into a small structure sufficiently to enter cells through endocytosis, and therefore they have been widely studied as a non-viral gene delivery system.

Nucleic acids such as antisense RNA, siRNA, and the like are a material capable of inhibiting expression of specific proteins in vivo, and are spotlighted as an important tool for treatment of cancer, genetic diseases, infectious diseases, autoimmune diseases, and the like (Novina and Sharp, Nature, 430, 161-164, 2004). However, nucleic acids such as siRNA are difficult to deliver directly into cells and they are easily decomposed by enzymes in the blood, so there are many studies to overcome them. To date, the method for delivering nucleic acids into cells, a method for carrying by mixing with a positive charge lipid or polymer (named lipid-DNA conjugate (lipoplex) and polymer-DNA conjugate (polyplex), respectively) is mainly used (Hirko et al., Curr. Med. Chem., 10, 1185-93, 2003; Merdan et al., Adv. Drug. Deliv. Rev., 54, 715-58, 2002; Spagnou et al., Biochemistry, 43, 13348-13356, 2004). The lipid-DNA conjugate is combined with the nucleic acid to deliver the nucleic acid well to cells and thus it is used a lot at the cell level, but in vivo, when injecting locally, in many cases, it has a disadvantage of inducing inflammation in the body (Filonand and Phillips, Biochim. Biophys. Acta, 1329, 345-56, 1997), and accumulating it in tissues such as lung, liver, spleen and the like which are mainly primary passage organs during intravascular injection (Ren et al., Gene Therapy, 7, 764-768, 2000).

In addition, such a non-viral delivery system has a problem of low transfection efficiency. Many efforts have been tilted to enhance transfection efficiency, but this is still far from the system that is stable. In addition, the carrier of the non-viral gene delivery system represents a significantly high cytotoxicity due to poor biocompatibility and non-biodegradability.

Under such a technical background, the present inventors have developed novel nanoparticles that can efficiently deliver an anionic drug, a nucleic acid, etc. by being specifically delivered to liver tissue, particularly LSEC.

[DISCLOSURE]

[TECHNICAL PROBLEM]

One embodiment provides a lipid nanoparticle comprising an ionizable lipid in which a 6-membered heterocyclic amine and an alkyl-epoxide are bonded, a phospholipid, a cholesterol, and a mixture of lipid-PEG (polyethyleneglycol) conjugates. The mixture of lipid-PEG conjugates may comprise a mannose-PEG-lipid conjugate.

Another object of the present invention is to provide a composition for delivering a drug (anionic drug, nucleic acid or a combination thereof) comprising (1) the lipid nanoparticle; and (2) an anionic drug, a nucleic acid, or a combination thereof.

Other object of the present invention is to provide a pharmaceutical composition for preventing or treating liver disease comprising (1) the lipid nanoparticle; and (2) an anionic drug, a nucleic acid, or a combination thereof.

[TECHNICAL SOLUTION]

One aspect to achieve the above object provides a lipid nanoparticle comprising an ionizable lipid in which a 6-membered heterocyclic amine and an alkyl-epoxide are bonded, a phospholipid, a cholesterol, and a mixture of lipid-PEG (polyethyleneglycol) conjugates, and

wherein the mixture of lipid-PEG conjugates comprises a mannose-PEG-lipid conjugate.

The lipid nanoparticle according to one embodiment is specific to liver tissue and/or LSEC (liver sinusoidal endothelial cell), and has excellent biocompatibility, and can deliver a gene therapeutic agent and the like with high efficiency, and thus it can be usefully used in related technical fields such as lipid nanoparticle-mediated gene therapy and image diagnosis technology.

Herein, ‘ionizable lipid’ or ’lipidoid’ mean an amine-containing lipid which can be easily protonated, and for example, it may be a lipid of which charge state changes depending on the surrounding pH. The ionizable lipid may be one in which a 6-membered heterocyclic amine and alkyl-epoxide are combined. Specifically, the ionizable lipid may be a compound having characteristics similar to the lipid produced by reaction of the 6-membered heterocyclic amine and alkyl-epoxide, and more specifically, it may be a compound produced by ring opening reaction of epoxide by reacting the 6-membered heterocyclic amine with alkyl-epoxide.

In one embodiment, the ionizable lipid may be one in which a 6-membered heterocyclic amine and alkyl-epoxide are combined by reacting them at a molar ratio of 1:n (n = number of primary amines comprised in 6-membered heterocyclic amine x 2 + number of secondary amines x 1). According to one specific embodiment, it may be prepared by mixing 246 amine and 1,2-epoxydodecane at a molar ratio of 1 : 4 and reacting them under the conditions of 700 to 800 rpm and 85 to 95 for 2 to 4 days.

The ionizable lipid may be protonated (positively charged) at a pH below the pKa of a cationic lipid, and it may be substantially neutral at a pH over the pKa. In one embodiment, the lipid nanoparticle may comprise a protonated ionizable lipid and/or an ionizable lipid showing neutrality.

The ionizable lipid is an ionizable compound having characteristics similar to the lipid, and through electrostatic interaction with a drug (for example, anionic drug and/or nucleic acid), may play a role of encapsulating the drug within the lipid nanoparticle with high efficiency.

The 6-membered heterocyclic amine may comprise a structure of piperazine or piperidine.

The 6-membered heterocyclic amine may be a chain or non-chain amine comprising a tertiary amine, and according to one embodiment, it may be one or more kinds selected from the group consisting of

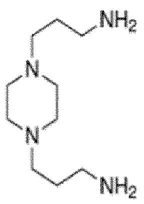 , 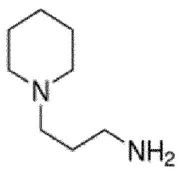 , 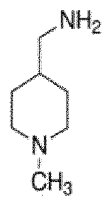 , 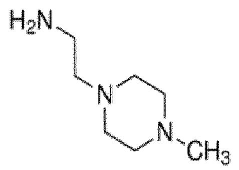 ,

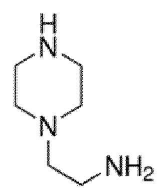 , and 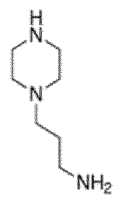 .

In one embodiment, the 6-membered heterocyclic amine may be one or more kinds selected from the group consisting of 1,4-bis(3-aminopropyl)piperazine, N-(3-Aminopropyl)piperidine, (1-Methyl-4-piperidinyl)methanamine, 2-(4-Methyl-piperazin-1-yl)-ethylamine, 1-(2-Aminoethyl)piperazine, and 1-(3-aminopropyl)piperazine.

According to the type of the amine comprised in the ionizable lipid, (i) the drug encapsulation efficiency, (ii) PDI (polydispersity index). and/or (iii) the drug delivery efficiency to liver tissue and/or LSEC (liver sinusoidal endothelial cell) of the lipid nanoparticle may be different.

The lipid nanoparticle comprising an ionizable lipid comprising an amine may have one or more kinds of the following characteristics:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or LSEC; and/or
(4) excellent targeting effect to LSEC (for example, it has a better targeting effect to LSEC than hepatocyte).

According to one embodiment, the lipid nanoparticle comprising an ionizable lipid comprising 1,4-bis(3-aminopropyl)piperazine (Cas Nos. 7209-38-3) may have one or more kinds of the following characteristics than that comprising an ionizable lipid comprising other types of amines:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells; and/or
(4) excellent targeting effect to LSEC (for example, it has a better targeting effect to LSEC than hepatocyte).

The alkyl-epoxide may have the structure of Chemical formula 1 below.

[Chemical formula 1]

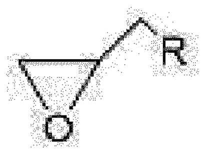

The alkyl-epoxide may have a carbon length of C6 to C14, C6 to C12, C6 to C10, C8 to C14, C8 to C12, C8 to C10, C10 to C14, C10 to C12, or C10, and for example, it may be 1,2-epoxydodecane of C10. According to one embodiment, the lipid nanoparticle comprising by setting the carbon number of the alkyl-epoxide comprised in the ionizable lipid to the above range, the lipid nanoparticle may have one or more kinds of the following characteristics:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells; and/or
(4) excellent targeting effect to LSEC (for example, it has a better targeting effect to LSEC than hepatocyte).

In one embodiment, the ionizable lipid may have the general formula of Chemical formula 2 below.

[Chemical formula 2]

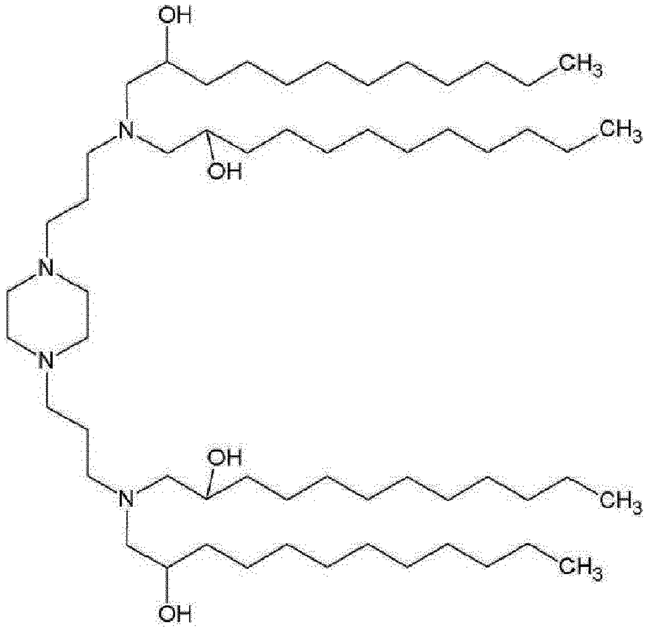

The structure of Chemical formula 2 is one example of the structure of the ionizable lipid according to one embodiment, and the structure of the ionizable lipid may be different depending on the type of the 6-membered heterocyclic amine and alkyl-epoxide.

According to one embodiment, a lipid nanoparticle comprising an ionizable lipid having the structure of Chemical formula 2 may have one or more kinds of the following characteristics than a lipid nanoparticle comprising other types of ionizable lipids:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or LSEC; and/or
(4) excellent targeting effect to LSEC (for example, it has a better targeting effect to LSEC than hepatocyte).

According to one embodiment, the lipid nanoparticle may have a pKa of 5 to 8, 5.5 to 7.5, 6 to 7, or 6.5 to 7. The pKa is an acid dissociation constant, and refers to what is generally used as an index indicating the acid strength of a target substance. The pKa value of the lipid nanoparticle is important in terms of in vivo stability of the lipid nanoparticle and drug release of the lipid nanoparticle. In one embodiment, the lipid nanoparticle showing the pKa value in the above range may be safely delivered to cancer tissue and/or cancer cells in vivo, and reach to the cancer tissue and/or cancer cells, and after endocytosis, exhibit a positive charge to release an encapsulated drug (the first anticancer agent) through electrostatic interaction with an anionic protein of the endosome membrane.

The phospholipid of the elements of the lipid nanoparticle according to one embodiment plays a role of covering and protecting a core formed by interaction of the ionizable lipid and drug (the first anticancer agent) in the lipid nanoparticle, and may facilitate cell membrane permeation and endosomal escape during intracellular delivery of the drug by binding to the phospholipid bilayer of a target cell.

For the phospholipid, a phospholipid which can promote fusion of the lipid nanoparticle according to one embodiment may be used without limitation, and for example, it may be one or more kinds selected from the group consisting of dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), dipalmitoylphosphatidylethanolamine, 1,2-dioleoyl-sn-glycero-3-phosphoethanolamine, 1-palmitoyl-2-oleoyl-sn-glycero-3-phosphoethanolamine (POPE), 1-palmitoyl-2-oleoyl-sn-glycero-3-phosphocholine (POPC), 1,2-dioleoyl-sn-glycero-3-[phospho-L-serine] (DOPS), 1,2-dioleoyl-sn-glycero-3-[phospho-L-serine] and the like. In one embodiment, the lipid nanoparticle comprising DOPE may be effective in mRNA delivery (excellent drug delivery efficiency to mRNA), and in other embodiment, the lipid nanoparticle comprising DSPE may be effective in siRNA delivery (excellent drug delivery efficiency to siRNA).

The cholesterol may provide morphological rigidity to lipid filling in the lipid nanoparticle and be dispersed in the core and surface of the nanoparticle to improve the stability of the nanoparticle.

Herein, "lipid-PEG (polyethyleneglycol) conjugate", "lipid-PEG", "PEG-lipid", "PEG-lipid", or "lipid-PEG" refers to a form in which lipid and PEG are conjugated, and may mean a lipid in which a polyethylene glycol (PEG) polymer which is a hydrophilic polymer is bound to one end.

Herein, "a mixture of lipid-PEG conjugate" may mean a mixture comprising various types of lipid-PEG conjugates. In one embodiment, the mixture of lipid-PEG conjugates may comprise mannose-PEG-lipid conjugate (or mannose-lipid-PEG conjugate) (for example, mannose-PEG-DSPE conjugate). In one embodiment, the mannose-PEG-lipid conjugate may be present in the out layer of the lipid nanoparticle.

The mixture of lipid-PEG conjugates may play a role in contributing to the particle stability in serum of the nanoparticle within the lipid nanoparticle, and plays a role of preventing aggregation between nanoparticles, and increasing the targeting effect to liver tissue and/or LSEC. In addition, the lipid-PEG conjugate may protect nucleic acids from degrading enzyme during in vivo delivery of the nucleic acids and enhance the stability of nucleic acids in vivo and increase the half-life of the drug encapsulated in the nanoparticle. In addition, the mixture of lipid-PEG conjugates can impart a stealth function to the nanoparticles in vivo to prevent degradation of the nanoparticles.

In the lipid-PEG conjugate, the PEG may be directly conjugated to the lipid or linked to the lipid via a linker moiety. Any linker moiety suitable for binding PEG to the lipid may be used, and for example, includes an ester-free linker moiety and an ester-containing linker moiety. The ester-free linker moiety includes not only amido (-C(O)NH-), amino (-NR-), carbonyl (-C(O)-), carbamate (-NHC(O)O-), urea (-NHC(O)NH-), disulfide (-S-S-), ether (-O-), succinyl (-(O)CCH2CH2C(O)-), succinamidyl (-NHC(O)CH2CH2C(O)NH-), ether, disulfide but also combinations thereof (for example, a linker containing both a carbamate linker moiety and an amido linker moiety), but not limited thereto. The ester-containing linker moiety includes for example, carbonate (-OC(O)O-), succinoyl, phosphate ester (-O-(O)POH-O-), sulfonate ester, and combinations thereof, but not limited thereto.

In one embodiment, the average molecular weight of the lipid-PEG conjugate may be 100 to 10000 daltons, 200 to 10000 daltons, 500 to 10000 daltons, 1000 to 10000 daltons, 1500 to 10000 daltons, 2000 to 10000 daltons, 100 to 7500 daltons, 200 to 7500 daltons, 500 to 7500 daltons, 1000 to 7500 daltons, 1500 to 7500 daltons, 2000 to 7500 daltons, 100 to 5000 daltons, 200 to 5000 daltons, 500 to 5000 daltons, 1000 to 5000 daltons, 1500 to 5000 daltons, 2000 to 5000 daltons, 100 to 3000 daltons, 200 to 3000 daltons, 500 to 3000 daltons, 1000 to 3000 daltons, 1500 to 3000 daltons, 2000 to 3000 daltons, 100 to 2600 daltons, 200 to 2600 daltons, 500 to 2600 daltons, 1000 to 2600 daltons, 1500 to 2600 daltons, 2000 to 2600 daltons, 100 to 2500 daltons, 200 to 2500 daltons, 500 to 2500 daltons, 1000 to 2500 daltons, 1500 to 2500 daltons, or 2000 to 2500 daltons.

For the lipid in the lipid-PEG conjugate, any lipid capable of binding to polyethyleneglycol may be used without limitation, and the phospholipid and/or cholesterol which are other elements of the lipid nanoparticle may be also used. Specifically, the lipid in the lipid-PEG conjugate may be ceramide, dimyristoylglycerol (DMG), succinoyl-diacylglycerol (s-DAG), distearoylphosphatidylcholine (DSPC), distearoylphosphatidylethanolamine (DSPE), or cholesterol.

In one embodiment, the lipid-PEG conjugate may comprise PEG bound to dialkyloxypropyl (PEG-DAA), PEG bound to diacylglycerol (PEG-DAG), PEG bound to a phospholipid such as phosphatidylethanolamine (PEG-PE), PEG conjugated to ceramide (PEG-CER, or ceramide-PEG conjugate), cholesterol or PEG conjugated to derivative thereof, PEG-c-DOMG, PEG-DMG, PEG-DLPE, PEG-DMPE, PEG-DPPC, PEG-DSPE, and a mixture thereof, in addition to the mannose-PEG-lipid conjugate, and for example, may be C16-PEG2000 ceramide, DMG-PEG 2000, 14:0 PEG2000 PE.

In one embodiment, the mixture of lipid-PEG conjugates may comprise a ceramide-PEG conjugate and/or a mannose-PEG-lipid conjugate (for example, a mannose-PEG-DSPE conjugate).

According to one embodiment, the case of comprising a lipid nanoparticle comprising a ceramide-PEG conjugate and/or a mannose-PEG-lipid conjugate (for example, a mannose-PEG-DSPE conjugate) may have one or more kinds of the following characteristics than the case of comprising a lipid nanoparticle comprising other types of lipid-PEG conjugates:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells; and/or
(4) excellent targeting effect to LSEC (for example, it has a better targeting effect to LSEC than hepatocyte).

The PEG in the lipid-PEG conjugate is a hydrophilic polymer and has an ability to inhibit adsorption of serum proteins, thereby increasing the circulation time of lipid nanoparticles in the body.

The PEG may be what a functional group binds to a side not bound to a lipid (functionalized PEG). In this case, the functional group that can be used may be one or more kinds selected from the group consisting of succinyl group, carboxylic acid, maleimide, amine group, biotin, cyanur group and folate, and the like.

According to one embodiment, the mixture of lipid-PEG conjugates may be comprised in the lipid nanoparticle

in an amount of 0.1 to 15 mol%, 0.25 to 15 mol%, 0.5 to 15 mol%, 1 to 15 mol%, 1.5 to 15 mol%, 2 to 15 mol%, 2.5 to 15 mol%, 3 to 15 mol%, 0.1 to 10 mol%, 0.25 to 10 mol%, 0.5 to 10 mol%, 1 to 10 mol%, 1.5 to 10 mol%, 2 to 10 mol%, 2.5 to 10 mol%, 3 to 10 mol%, 0.1 to 9 mol%, 0.25 to 9 mol%, 0.5 to 9 mol%, 1 to 9 mol%, 1.5 to 9 mol%, 2 to 9 mol%, 2.5 to 9 mol%, 3 to 9 mol%, 0.1 to 7.5 mol%, 0.25 to 7.5 mol%, 0.5 to 7.5 mol%, 1 to 7.5 mol%, 1.5 to 7.5 mol%, 2 to 7.5 mol%, 2.5 to 7.5 mol%, 3 to 7.5 mol%, 0.1 to 5 mol%, 0.25 to 5 mol%, 0.5 to 5 mol%, 1 to 5 mol%, 1.5 to 5 mol%, 2 to 5 mol%, 2.5 to 5 mol%, 3 to 5 mol%, 0.1 to 4.5 mol%, 0.25 to 4.5 mol%, 0.5 to 4.5 mol%, 1 to 4.5 mol%, 1.5 to 4.5 mol%, 2 to 4.5 mol%, 2.5 to 4.5 mol%, 3 to 4.5 mol%, 0.1 to 4 mol%, 0.25 to 4 mol%, 0.5 to 4 mol%, 1 to 4 mol%, 1.5 to 4 mol%, 2 to 4 mol%, 2.5 to 4 mol%, 3 to 4 mol%, 0.1 to 3.5 mol%, 0.25 to 3.5 mol%, 0.5 to 3.5 mol%, 1 to 3.5 mol%, 1.5 to 3.5 mol%, 2 to 3.5 mol%, 2.5 to 3.5 mol%, 3 to 3.5 mol%, 0.1 to 3 mol%, 0.25 to 3 mol%, 0.5 to 3 mol%, 1 to 3 mol%, 1.5 to 3 mol%, 2 to 3 mol%, or 2.5 to 3 mol%.

According to one embodiment, the mannose-PEG lipid conjugate may be comprised in the lipid nanoparticle in an amount of 0.1 to 10 mol%, 0.25 to 10 mol%, 0.5 to 10 mol%, 1 to 10 mol%, 1.5 to 10 mol%, 2 to 10 mol%, 0.1 to 7.5 mol%, 0.25 to 7.5 mol%, 0.5 to 7.5 mol%, 1 to 7.5 mol%, 1.5 to 7.5 mol%, 2 to 7.5 mol%, 0.1 to 5 mol%, 0.25 to 5 mol%, 0.5 to 5 mol%, 1 to 5 mol%, 1.5 to 5 mol%, 2 to 5 mol%, 0.1 to 4.5 mol%, 0.25 to 4.5 mol%, 0.5 to 4.5 mol%, 1 to 4.5 mol%, 1.5 to 4.5 mol%, 2 to 4.5 mol%, 0.1 to 4 mol%, 0.25 to 4 mol%, 0.5 to 4 mol%, 1 to 4 mol%, 1.5 to 4 mol%, 2 to 4 mol%, 0.1 to 3 mol%, 0.25 to 3 mol%, 0.5 to 3 mol%, 1 to 3 mol%, 1.5 to 3 mol%, 2 to 3 mol%, 0.1 to 2.5 mol%, 0.25 to 2.5 mol%, 0.5 to 2.5 mol%, 1 to 2.5 mol%, 1.5 to 2.5 mol%, 2 to 2.5 mol%, 0.1 to 2 mol%, 0.25 to 2 mol%, 0.5 to 2 mol%, 1 to 2 mol%, or 1.5 to 2 mol%.

In one embodiment, the mixture of lipid-PEG conjugates may comprise other types of lipid-PEG conjugate other than the mannose-PEG-lipid conjugate, and the other types of lipid-PEG conjugate other than the mannose-PEG-lipid conjugate may be comprised in the lipid nanoparticle in an amount of 0.1 to 10 mol%, 0.25 to 10 mol%, 0.5 to 10 mol%, 1 to 10 mol%, 1.5 to 10 mol%, 2 to 10 mol%, 0.1 to 7.5 mol%, 0.25 to 7.5 mol%, 0.5 to 7.5 mol%, 1 to 7.5 mol%, 1.5 to 7.5 mol%, 2 to 7.5 mol%, 0.1 to 5 mol%, 0.25 to 5 mol%, 0.5 to 5 mol%, 1 to 5 mol%, 1.5 to 5 mol%, 2 to 5 mol%, 0.1 to 4.5 mol%, 0.25 to 4.5 mol%, 0.5 to 4.5 mol%, 1 to 4.5 mol%, 1.5 to 4.5 mol%, 2 to 4.5 mol%, 0.1 to 4 mol%, 0.25 to 4 mol%, 0.5 to 4 mol%, 1 to 4 mol%, 1.5 to 4 mol%, 2 to 4 mol%, 0.1 to 3 mol%, 0.25 to 3 mol%, 0.5 to 3 mol%, 1 to 3 mol%, 1.5 to 3 mol%, 2 to 3 mol%, 0.1 to 2.5 mol%, 0.25 to 2.5 mol%, 0.5 to 2.5 mol%, 1 to 2.5 mol%, 1.5 to 2.5 mol%, 2 to 2.5 mol%, 0.1 to 2 mol%, 0.25 to 2 mol%, 0.5 to 2 mol%, 1 to 2 mol%, 1.5 to 2 mol%, 0.1 to 5 mol%, 0.25 to 5 mol%, 0.3 to 5 mol%, 0.4 to 5 mol%, 0.5 to 5 mol%, 0.1 to 4 mol%, 0.25 to 4 mol%, 0.3 to 4 mol%, 0.4 to 4 mol%, 0.5 to 4 mol%, 0.1 to 3.5 mol%, 0.25 to 3.5 mol%, 0.3 to 3.5 mol%, 0.4 to 3.5 mol%, 0.5 to 3.5 mol%, 0.1 to 3 mol%, 0.25 to 3 mol%, 0.3 to 3 mol%, 0.4 to 3 mol%, 0.5 to 3 mol%, 0.1 to 2.5 mol%, 0.25 to 2.5 mol%, 0.3 to 2.5 mol%, 0.4 to 2.5 mol%, 0.5 to 2.5 mol%, 0.1 to 2 mol%, 0.25 to 2 mol%, 0.3 to 2 mol%, 0.4 to 2 mol%, 0.5 to 2 mol%, 0.1 to 1.5 mol%, 0.25 to 1.5 mol%, 0.3 to 1.5 mol%, 0.4 to 1.5 mol%, 0.5 to 1.5 mol%, 0.1 or more, to 1 mol%, 0.25 or more, to 1 mol%, 0.3 or more, to 1 mol%, 0.4 or more, to 1 mol%, 0.5 or more, to 1 mol%, 0.1 or more, to 1 less mol%, 0.25 or more, to 1 less mol%, 0.3 or more, to 1 less mol%, 0.4 or more, to 1 less mol%, 0.5 or more, to 1 less mol%, 0.1 to 0.75 mol%, 0.25 to 0.75 mol%, 0.3 to 0.75 mol%, 0.4 to 0.75 mol%, 0.5 to 0.75 mol%, 0.1 to 0.5 mol%, 0.25 to 0.5 mol%, 0.3 to 0.5 mol%, 0.4 to 0.5 mol%, or 0.5 to 0.5 mol%.

The lipid nanoparticle comprising the mixture of the lipid-PEG conjugates, the mannose-PEG-lipid conjugate, and/or the lipid-PEG conjugate other than mannose-PEG-lipid conjugates (for example, ceramide-PEG conjugate) in the above range (rather than the lipid nanoparticle comprising a mixture of lipid-PEG conjugates, mannose-PEG-lipid conjugate, and/or the lipid-PEG conjugate other than mannose-PEG-lipid conjugate in an amount outside the above range) may have one or more kinds of the following characteristics:

(1) encapsulating a drug into the lipid nanoparticle with high efficiency;
(2) uniform size of the prepared lipid nanoparticle (or having a low PDI value); and/or
(3) excellent drug delivery efficiency to cancer tissue, and/or cancer cells; and/or
(4) excellent targeting effect to LSEC (for example, better targeting effect to LSEC than hepatocyte).

According to one embodiment, the cholesterol may be comprised in the lipid nanoparticle in an amount of 10 to 60 mol%, 20 to 60 mol%, 30 to 60 mol%, 34.5 to 60 mol%, 35 to 60 mol%, 39.5 to 60 mol%, 40 to 60 mol%, 43 to 60 mol%, 44 to 60 mol%, 10 to 55 mol%, 20 to 55 mol%, 30 to 55 mol%, 34.5 to 55 mol%, 35 to 55 mol%, 39.5 to 55 mol%, 40 to 55 mol%, 43 to 55 mol%, 44 to 55 mol%, 10 to 52.5 mol%, 20 to 52.5 mol%, 30 to 52.5 mol%, 34.5 to 52.5 mol%, 35 to 52.5 mol%, 39.5 to 52.5 mol%, 40 to 52.5 mol%, 43 to 52.5 mol%, 44 to 52.5 mol%, 10 to 51 mol%, 20 to 51 mol%, 30 to 51 mol%, 34.5 to 51 mol%, 35 to 51 mol%, 39.5 to 51 mol%, 40 to 51 mol%, 43 to 51 mol%, 44 to 51 mol%, 10 to 50 mol%, 20 to 50 mol%, 30 to 50 mol%, 34.5 to 50 mol%, 35 to 50 mol%, 39.5 to 50 mol%, 40 to 50 mol%, 43 to 50 mol%, 44 to 50 mol%, 10 to 45 mol%, 20 to 45 mol%, 30 to 45 mol%, 34.5 to 45 mol%, 35 to 45 mol%, 39.5 to 45 mol%, 40 to 45 mol%, 43 to 45 mol%, 44 to 45 mol%, 10 to 44.25 mol%, 20 to 44.25 mol%, 30 to 44.25 mol%, 34.5 to 44.25 mol%, 35 to 44.25 mol%, 39.5 to 44.25 mol%, 40 to 44.25 mol%, 43 to 44.25 mol%, 44 to 44.25 mol%, 10 to 44 mol%, 20 to 44 mol%, 30 to 44 mol%, 34.5 to 44 mol%, 35 to 44 mol%, 39.5 to 44 mol%, 40 to 44 mol%, 43 to 44 mol%, 44 to

44 mol%, 10 to 43 mol%, 20 to 43 mol%, 30 to 43 mol%, 34.5 to 43 mol%, 35 to 43 mol%, 39.5 to 43 mol%, 40 to 43 mol%, 30 to 60 mol%, 30 to 55 mol%, 30 to 52.5 mol%, 30 to 52 mol%, 30 to 51 mol%, 30 to 50 mol%, 30 to 47.5 mol%, 30 to 45 mol%, 30 to 44 mol%, 30 to 43.5 mol%, 30 to 43 mol%, 30 to 41.5 mol%, 30 to 40 mol%, 30 to 39.5 mol%, 35 to 60 mol%, 35 to 55 mol%, 35 to 52.5 mol%, 35 to 52 mol%, 35 to 51 mol%, 35 to 50 mol%, 35 to 47.5 mol%, 35 to 45 mol%, 35 to 44 mol%, 35 to 43.5 mol%, 35 to 43 mol%, 35 to 41.5 mol%, 35 to 40 mol%, 35 to 39.5 mol%, 37 to 60 mol%, 37 to 55 mol%, 37 to 52.5 mol%, 37 to 52 mol%, 37 to 51 mol%, 37.5 to 50 mol%, 37.5 to 47.5 mol%, 37.5 to 45 mol%, 37.5 to 44 mol%, 37.5 to 43.5 mol%, 37.5 to 43 mol%, 37.5 to 41.5 mol%, 37.5 to 40 mol%, 37.5 to 39.5 mol%, 39.5 to 60 mol%, 39.5 to 55 mol%, 39.5 to 52.5 mol%, 39.5 to 52 mol%, 39.5 to 51 mol%, 39.5 to 50 mol%, 39.5 to 47.5 mol%, 39.5 to 45 mol%, 39.5 to 44 mol%, 39.5 to 43.5 mol%, 39.5 to 43 mol%, 39.5 to 41.5 mol%, 39.5 to 40 mol%, 40 to 60 mol%, 40 to 55 mol%, 40 to 52.5 mol%, 40 to 52 mol%, 40 to 51 mol%, 40 to 50 mol%, 40 to 47.5 mol%, 40 to 45 mol%, 40 to 44 mol%, 40 to 43.5 mol%, 40 to 43 mol%, 40 to 41.5 mol%, 41.5 to 60 mol%, 41.5 to 55 mol%, 41.5 to 52.5 mol%, 41.5 to 52 mol%, 41.5 to 51 mol%, 41.5 to 50 mol%, 41.5 to 47.5 mol%, 41.5 to 45 mol%, 41.5 to 44 mol%, 41.5 to 43.5 mol%, 41.5 to 43 mol%, 43 to 60 mol%, 43 to 55 mol%, 43 to 52.5 mol%, 43 to 52 mol%, 43 to 51 mol%, 43 to 50 mol%, 43 to 47.5 mol%, 43 to 45 mol%, 43 to 44 mol%, 43 to 43.5 mol%, 43.5 to 60 mol%, 43.5 to 55 mol%, 43.5 to 52.5 mol%, 43.5 to 52 mol%, 43.5 to 51 mol%, 43.5 to 50 mol%, 43.5 to 47.5 mol%, 43.5 to 45 mol%, 43.5 to 44 mol%, 45 to 60 mol%, 45 to 55 mol%, 45 to 52.5 mol%, 45 to 52 mol%, 45 to 51 mol%, 45 to 50 mol%, 45 to 47.5 mol%, 47.5 to 60 mol%, 47.5 to 55 mol%, 47.5 to 52.5 mol%, 47.5 to 52 mol%, 47.5 to 51 mol%, 47.5 to 50 mol%,%, 50 to 60 mol%, 50 to 55 mol%, 50 to 52.5 mol%, 50 to 52 mol%, 50 to 52.5 mol% 50 to 51.5 mol%, 51 to 60 mol%, 51 to 55 mol%, 51 to 52.5 mol%, or 51 to 52 mol%, 51 to 60 mol%, 51 to 55 mol%, 51 to 52.5 mol%, or 51 to 52 mol%.

According to one embodiment, the sum of the lipid-PEG conjugate and cholesterol may be comprised in the lipid nanoparticle in an amount of 40 to 60 mol%, 40 to 55 mol%, 40 to 53.5 mol%, 40 to 50 mol%, 40 to 47.5 mol%, 40 to 45 mol%, 40 to 44.5 mol%, 42 to 60 mol%, 42 to 55 mol%, 42 to 53.5 mol%, 42 to 50 mol%, 42 to 47.5 mol%, 42 to 45 mol%, 42 to 44,5 mol%, 44 to 60 mol%, 44 to 55 mol%, 44 to 53.5 mol%, 44 to 50 mol%, 44 to 47.5 mol%, 44 to 45 mol%, 44 to 44.5 mol%, 44.5 to 60 mol%, 44.5 to 55 mol%, 44.5 to 53.5 mol%, 44.5 to 50 mol%, 44.5 to 47.5 mol%, or 44.5 to 45 mol%.

According to one embodiment, the ionizable lipid may be comprised in the lipid nanoparticle in an amount of 10 to 60 mol%, 10 to 55 mol%, 10 to 50 mol%, 10 to 45 mol%, 10 to 42.5 mol%, 10 to 40 mol%, 10 to 35 mol%, 10 to 30 mol%, 10 to 26.5 mol%, 10 to 25 mol%, 10 to 20 mol%, 15 to 60 mol%, 15 to 55 mol%, 15 to 50 mol%, 15 to 45 mol%, 15 to 42.5 mol%, 15 to 40 mol%, 15 to 35 mol%, 15 to 30 mol%, 15 to 26.5 mol%, 15 to 25 mol%, 15 to 20 mol%, 20 to 60 mol%, 20 to 55 mol%, 20 to 50 mol%, 20 to 45 mol%, 20 to 42.5 mol%, 20 to 40 mol%, 20 to 35 mol%, 20 to 30 mol%, 20 to 26.5 mol%, 20 to 25 mol%, 25 to 60 mol%, 25 to 55 mol%, 25 to 50 mol%, 25 to 45 mol%, 25 to 42.5 mol%, 25 to 40 mol%, 25 to 35 mol%, 25 to 30 mol%, 25 to 26.5 mol%, 26.5 to 60 mol%, 26.5 to 55 mol%, 26.5 to 50 mol%, 26.5 to 45 mol%, 26.5 to 42.5 mol%, 26.5 to 40 mol%, 26.5 to 35 mol%, 26.5 to 30 mol%, 30 to 60 mol%, 30 to 55 mol%, 30 to 50 mol%, 30 to 45 mol%, 30 to 42.5 mol%, 30 to 40 mol%, 30 to 35 mol%, 35 to 60 mol%, 35 to 55 mol%, 35 to 50 mol%, 35 to 45 mol%, 35 to 42.5 mol%, 35 to 40 mol%, 40 to 60 mol%, 40 to 55 mol%, 40 to 50 mol%, 40 to 45 mol%, 40 to 42.5 mol%, 42.5 to 60 mol%, 42.5 to 55 mol%, 42.5 to 50 mol%, or 42.5 to 45 mol%.

According to one embodiment, the phospholipid may be comprised in the lipid nanoparticle in an amount of 5 to 30 mol%, 5 to 25 mol%, 5 to 20 mol%, 5 to 15 mol%, 5 to 13 mol%, 5 to 10 mol%, 10 to 30 mol%, 10 to 25 mol%, 10 to 20 mol%, 10 to 15 mol%, 10 to 13 mol%, 15 to 30 mol%, 15 to 25 mol%, 15 to 20 mol%, 20 to 30 mol%, or 20 to 25 mol%.

Herein, "mol% (mol%, mole percentage)" is expressed as a percentage by dividing the number of moles of a specific component by the sum of moles of all components and then multiplying by 100, and expressed as a formula, for example, it may be as Equation 1 below.

(Equation 1)

$$\text{mol\%} = (\text{moles of a specific component}) / (\text{sum of moles of all components}) \times 100$$

The lipid nanoparticle may comprise the ionizable lipid : phospholipid : cholesterol : a mixture of lipid-PEG conjugates at a molar ratio of 20 to 50 : 10 to 30 : 30 to 60 : 0.1 to 5, at a molar ratio of 20 to 50 : 10 to 30 : 30 to 60 : 0.5 to 5, at a molar ratio of 20 to 50 : 10 to 30 : 30 to 60 : 1.5 to 3, at a molar ratio of 25 to 45 : 10 to 25 : 40 to 50 : 0.5 to 3, at a molar ratio of 25 to 45 : 10 to 20 : 40 to 55 : 0.5 to 3, at a molar ratio of 25 to 45 : 10 to 20 : 40 to 55 : 1.0 to 1.5, at a molar ratio of 40 to 45 : 10 to 15 : 40 to 45 : 0.5 to 3.0, at a molar ratio of 40 to 45 : 10 to 15 : 40 to 45 : 0.5 to 3, at a molar ratio of 40 to 45 : 10 to 15 : 40 to 45 : 1 to 1.5, at a molar ratio of 25 to 30: 17 to 22; 50 to 55 : 0.5 to 3.0, at a molar ratio of 25 to 30: 17 to 22; 50 to 55 : 1.0 to 2.5, at a molar ratio of 25 to 30: 17 to 22; 50 to 55 : 1.5 to 2.5. while maintaining the sum of the moles of the mixture of lipid-PEG conjugates and cholesterol constant, among the components comprised in the lipid nanoparticle, the moles of cholesterol are decreased as much as the number of moles of the mixture of lipid-PEG conjugates is increased, and thereby the molar ratio of the components can be maintained.

Herein, the molar ratio means a ratio of moles.

The lipid nanoparticle may comprise an ionizable lipid of 20 to 50 parts by weight, a phospholipid of 10 to 30 parts by weight, cholesterol of 30 to 60 parts by weight, and a mixture of lipid-PEG conjugates of 0.1 to 5 parts by weight (or 0.5 to 5 parts by weight).

Herein, "parts by weight" means a weight ratio of each component comprised.

The lipid nanoparticle may comprise the ionizable lipid of 20 to 50 parts by weight, phospholipid of 10 to 30 parts by weight, cholesterol of 40 to 60 parts by weight, and a mixture of lipid-PEG conjugates of 1.5 to 3 parts by weight based on the total nanoparticle weight. Specifically, the lipid nanoparticle may comprise the ionizable lipid of 25 to 50 parts by weight, phospholipid of 10 to 20 parts by weight, cholesterol of 35 to 55 parts by weight, and a mixture of lipid-PEG conjugates of 0.5 to 5.0 parts by weight based on the total nanoparticle weight.

The lipid nanoparticle comprising an ionizable lipid, cholesterol, a phospholipid, and/or a mixture of lipid-PEG conjugates in the above range (range of molar ratio, part by weight, and/or % by weight) may have excellent (i) stability of the lipid nanoparticle; (ii) encapsulation efficiency of the anticancer agent; and/or (iii) drug delivery efficiency to liver tissue and/or LSEC, than the case of comprising the lipid nanoparticle comprising an ionizable lipid, cholesterol, a phospholipid and/or a mixture of lipid-PEG conjugates outside the above range.

The lipid nanoparticle according to one example may have an average diameter of 20nm to 200nm, 20 to 180nm, 20nm to 170nm, 20nm to 150nm, 20nm to 120nm, 20nm to 100nm, 20nm to 90nm, 30nm to 200nm, 30 to 180nm, 30nm to 170nm, 30nm to 150nm, 30nm to 120nm, 30nm to 100nm, 30nm to 90nm, 40nm to 200nm, 40 to 180nm, 40nm to 170nm, 40nm to 150nm, 40nm to 120nm, 40nm to 100nm, 40nm to 90nm, 50nm to 200nm, 50 to 180nm, 50nm to 170nm, 50nm to 150nm, 50nm to 120nm, 50nm to 100nm, 50nm to 90nm, 60nm to 200nm, 60 to 180nm, 60nm to 170nm, 60nm to 150nm, 60nm to 120nm, 60nm to 100nm, 60nm to 90nm, 70nm to 200nm, 70 to 180nm, 70nm to 170nm, 70nm to 150nm, 70nm to 120nm, 70nm to 100nm, 70nm to 90nm, 80nm to 200nm, 80 to 180nm, 80nm to 170nm, 80nm to 150nm, 80nm to 120nm, 80nm to 100nm, 80nm to 90nm, 90nm to 200nm, 90 to 180nm, 90nm to 170nm, 90nm to 150nm, 90nm to 120nm, or 90nm to 100nm, for easy introduction into liver tissue, and/or LSEC (liver sinusoidal endothelial cells). When the size of the lipid nanoparticle is smaller than the above range, it is difficult to maintain stability as the surface area of the lipid nanoparticle is excessively increased, and thus delivery to the target tissue and/or drug effect may be reduced.

In one embodiment, in case of the lipid nanoparticle having a diameter of 20 to 200nm, 20to 180 nm, 40 to 180nm, 40 to 170nm, 50 to 160nm, 70 to 180nm, 70 to 170nm, 75 to 170nm, 75 to 165nm, 70 to 150nm, 70 to 130nm, 75 to 130nm, 80 to 120nm, 85 to 120nm, about 90 to 120nm, 90 to 110nm, 90 to 100nm, 80 to 110nm, 80 to 100nm, 85 to 95nm, about 90nm, or 90nm, the targeting effect to LSEC may be excellent (than the nanoparticle having a diameter outside the above range).

The lipid nanoparticle may specifically target liver tissue. The lipid nanoparticle according to one example may imitate metabolic behaviors of natural lipoproteins very similarly, and may be usefully applied for the lipid metabolism process by the liver and therapeutic mechanism through this.

The lipid nanoparticle may target an LSEC (liver sinusoidal endothelial cell). When the content of the mixture of lipid-PEG conjugates comprised in the lipid nanoparticle is 0.1 to 15 mol%, 0.25 to 15 mol%, 0.5 to 15 mol%, 1 to 15 mol%, 1.5 to 15 mol%, 2 to 15 mol%, 2.5 to 15 mol%, 3 to 15 mol%, 0.1 to 10 mol%, 0.25 to 10 mol%, 0.5 to 10 mol%, 1 to 10 mol%, 1.5 to 10 mol%, 2 to 10 mol%, 2.5 to 10 mol%, 3 to 10 mol%, 0.1 to 9 mol%, 0.25 to 9 mol%, 0.5 to 9 mol%, 1 to 9 mol%, 1.5 to 9 mol%, 2 to 9 mol%, 2.5 to 9 mol%, 3 to 9 mol%, 0.1 to 7.5 mol%, 0.25 to 7.5 mol%, 0.5 to 7.5 mol%, 1 to 7.5 mol%, 1.5 to 7.5 mol%, 2 to 7.5 mol%, 2.5 to 7.5 mol%, 3 to 7.5 mol%, 0.1 to 5 mol%, 0.25 to 5 mol%, 0.5 to 5 mol%, 1 to 5 mol%, 1.5 to 5 mol%, 2 to 5 mol%, 2.5 to 5 mol%, 3 to 5 mol%, 0.1 to 4.5 mol%, 0.25 to 4.5 mol%, 0.5 to 4.5 mol%, 1 to 4.5 mol%, 1.5 to 4.5 mol%, 2 to 4.5 mol%, 2.5 to 4.5 mol%, 3 to 4.5 mol%, 0.1 to 4 mol%, 0.25 to 4 mol%, 0.5 to 4 mol%, 1 to 4 mol%, 1.5 to 4 mol%, 2 to 4 mol%, 2.5 to 4 mol%, 3 to 4 mol%, 0.1 to 3.5 mol%, 0.25 to 3.5 mol%, 0.5 to 3.5 mol%, 1 to 3.5 mol%, 1.5 to 3.5 mol%, 2 to 3.5 mol%, 2.5 to 3.5 mol%, 3 to 3.5 mol%, 0.1 to 3 mol%, 0.25 to 3 mol%, 0.5 to 3 mol%, 1 to 3 mol%, 1.5 to 3 mol%, 2 to 3 mol%, or 2.5 to 3 mol%, the drug delivery efficiency (LSEC targeting efficiency) to LSEC of the lipid nanoparticle may be excellent.

Herein, "targeting" and "localization" to the liver tissue, and/or LSEC of the lipid nanoparticle may be internalization in the tissue or cells, and may mean internalization inside the nucleus as it can penetrate the nuclear membrane.

As other aspect, a composition for drug delivery comprising (i) the lipid nanoparticle; and (ii) an anionic drug, nucleic acid or combination thereof (combination of the anionic drug and nucleic acid) is provided. The drug may be an anionic drug, nucleic acid or combination thereof (anionic drug and nucleic acid).

The composition for drug delivery may be what a bioactive substance such as an anionic drug and/or nucleic acid, and the like may be encapsulated inside the lipid nanoparticle, and the bioactive substance such as an anionic drug and/or nucleic acid may be encapsulated with stable and high efficiency and thereby, it may show an excellent therapeutic effect by the composition for delivery. In addition, there is an advantage of variously controlling the kinds of the drug to be encapsulated in the lipid nanoparticle according to the purpose of treatment.

The lipid nanoparticle may have an anionic drug and/or nucleic acid encapsulated inside (the lipid nanoparticle).

For the lipid nanoparticle in which an anionic drug and/or nucleic acid is encapsulated (inside the lipid nanoparticle) is the same as for the lipid nanoparticle described above.

In one embodiment, the weight ratio of the ionizable lipid and drug (anionic drug, nucleic acid or combination thereof) comprised in the lipid nanoparticle may be 1 to 20 : 1, 1 to 15 : 1, 1 to 10 : 1, 5 to 20 : 1, 5 to 15 : 1, 5 to 10 : 1, 7.5 to 20 : 1, 7.5 to 15 : 1, or 7.5 to 10 : 1.

In one embodiment, when the (i) ionizable lipid; and (2) drug (anionic drug, nucleic acid or combination thereof) are comprised at a weight ratio in the above range, the encapsulation efficiency of the drug (anionic drug, nucleic acid or combination thereof) inside the lipid nanoparticle and/or drug delivery efficiency may be higher than the lipid nanoparticle comprising the (1) ionizable lipid; and (2) anionic drug, nucleic acid or combination thereof at a weight ratio outside the above range.

In one embodiment, the composition may specifically deliver a drug to CD206 positive cells and/or LSEC (liver sinusoidal endothelial cell).

The lipid nanoparticle in which the anionic drug and/or nucleic acid is encapsulated may have an average diameter of 20nm to 200nm, 20 to 180nm, 20nm to 170nm, 20nm to 150nm, 20nm to 120nm, 20nm to 100nm, 20nm to 90nm, 30nm to 200nm, 30 to 180nm, 30nm to 170nm, 30nm to 150nm, 30nm to 120nm, 30nm to 100nm, 30nm to 90nm, 40nm to 200nm, 40 to 180nm, 40nm to 170nm, 40nm to 150nm, 40nm to 120nm, 40nm to 100nm, 40nm to 90nm, 50nm to 200nm, 50 to 180nm, 50nm to 170nm, 50nm to 150nm, 50nm to 120nm, 50nm to 100nm, 50nm to 90nm, 60nm to 200nm, 60 to 180nm, 60nm to 170nm, 60nm to 150nm, 60nm to 120nm, 60nm to 100nm, 60nm to 90nm, 70nm to 200nm, 70 to 180nm, 70nm to 170nm, 70nm to 150nm, 70nm to 120nm, 70nm to 100nm, 70nm to 90nm, 80nm to 200nm, 80 to 180nm, 80nm to 170nm, 80nm to 150nm, 80nm to 120nm, 80nm to 100nm, 80nm to 90nm, 90nm to 200nm, 90 to 180nm, 90nm to 170nm, 90nm to 150nm, 90nm to 120nm, or 90nm to 100nm, so that the introduction into the liver tissue, CD206 positive cells, and/or LSEC (liver sinusoidal endothelial cells) is easy.

When the size of the lipid nanoparticle is smaller than the lower limit of the above range, (i) during systemic circulation of the lipid nanoparticle, binding of apolipoproteins (for example, ApoE (e.g. ApoE3)) present in blood is reduced, and therefore, the number of the lipid nanoparticles entering the cells may be reduced and/or (ii) the surface area of the lipid nanoparticle is excessively increased and therefore it is difficult to maintain the stability, and thus the drug delivery efficiency to target tissue (or target cell) and/or therapeutic effect of the drug which the lipid nanoparticle carries out may be reduced.

The lipid nanoparticle having a diameter in the above range may have excellent drug delivery efficiency to a target organ and/or cell than the lipid nanoparticle having a diameter over the upper limit of the above range.

According to one embodiment, the lipid nanoparticle comprised in the composition for drug delivery into LSEC may comprise a mixture of the lipid-PEG conjugates, a mannose-PEG-lipid conjugate, and/or a lipid-PEG conjugate other than mannose-PEG-lipid conjugates (for example, ceramide-PEG conjugate) in the above range, and the lipid nanoparticle comprising a mixture of the lipid-PEG conjugates, a mannose-PEG-lipid conjugate, and/or a lipid-PEG conjugate other than mannose-PEG-lipid conjugates may have excellent drug delivery efficiency specific to LSEC (or targeting LSEC).

According to one example, the nanoparticle comprised in the composition for drug delivery into LSEC comprising an ionizable lipid, cholesterol, a phospholipid, and/or a mixture of lipid-PEG conjugates in the above range (range of molar ratio, part by weight, and/or % by weight) may have excellent drug delivery efficiency specific to LSEC (or targeting LSEC), than the case of comprising the lipid nanoparticle comprising an ionizable lipid, cholesterol, a phospholipid and/or a mixture of lipid-PEG conjugates outside the above range.

The lipid nanoparticle according to one embodiment may delivery a therapeutic agent highly efficiently into liver tissue, and/or LSEC through liver tissue-specific properties, and may be usefully utilized for a drug for treating liver diseases, a delivery method of a gene for treatment, and the like, and a therapeutic agent mediated by liver, which mediate this highly efficient liver tissue, and/or LSEC-specific lipid nanoparticle. In addition, the lipid nanoparticle according to one embodiment forms a stable complex with a gene drug such as nucleic acid, and the like, and shows low cytotoxicity and effective cell absorption, and thus it is effective to deliver the gene drug such as nucleic acid.

The lipid nanoparticle is same as described above.

The lipid nanoparticle according to one embodiment exhibits a positive charge under the acidic pH condition by showing a pKa of 5 to 8, 5.5 to 7.5, 6 to 7, or 6.5 to 7, and may encapsulate a drug with high efficiency by easily forming a complex with a drug through electrostatic interaction with a therapeutic agent such as a nucleic acid and anionic drug (for example, protein) showing a negative charge, and it may be usefully used as a composition for intracellular or in vivo drug delivery of a drug (for example, nucleic acid).

Herein, "encapsulation" refers to encapsulating a delivery substance for surrounding and embedding it in vivo efficiently, and the drug encapsulation rate (encapsulation efficiency) mean the content of the drug encapsulated in the lipid nanoparticle for the total drug content used for preparation.

The encapsulation efficiency of the anionic or nucleic acid drug of the composition for delivery may be 70% or more, 75% or more, 80% or more, 85% or more, 90% or more, 91% or more, 92% or more, 94% or more, over 80% to

99% or less, over 80% to 97% or less, over 80% to 95% or less, 85% or more to 95% or less, 87% or more to 95% or less, 90% or more to 95% or less, 91% or more to 95% or less, 91% or more to 94% or less, over 91% to 95% or less, 92% or more to 99% or less, 92% or more to 97% or less, or 92% or more to 95% or less.

According to one embodiment, the encapsulation efficiency may be calculated by commonly used methods, and for example, the drug encapsulation efficiency may be calculated by the following Equation 2, by treating Triton-X to the lipid nanoparticle according to one example and measuring the fluorescence intensity of the Triton-X-treated and Triton-X-untreated lipid nanoparticles in a specific wavelength bandwidth (for example, excitation: 480 ~ 490nm, emission: 520 ~ 530nm).

(Equation 2)

Drug encapsulation efficiency (%) = (Fluorescence intensity (fluorescence) of the Triton-X-treated lipid nanoparticle - Fluorescence intensity (fluorescence) of the Triton-X-untreated lipid nanoparticle)/(Fluorescence intensity (fluorescence) of the Triton-X-treated lipid nanoparticle) X 100

The composition for drug delivery according to one embodiment may comprise Cas9 mRNA with high encapsulation efficiency. The previously known composition for delivering Cas9 mRNA has a limitation in using it as a composition for delivering Cas9 mRNA, as it comprises Cas9 mRNA at a low ratio. On the other hand, the lipid nanoparticle according to one example may comprise Cas9 mRNA with high encapsulation efficiency, specifically, encapsulation efficiency of 70% or more, and thus it may be usefully utilized for gene editing therapy.

The anionic drug may be an anionic biopolymer-drug conjugate such as various kinds of peptides, protein drugs, protein-nucleic acid structures or hyaluronic acid-peptide conjugates, hyaluronic acid-protein conjugates, which have an anion, and the like. The non-restrictive examples of the protein drugs may be apoptosis-inducing factors (e.g., cytocrome C, caspase 3/7/8/9, etc.) and including gene editing proteins such as Cas 9, cpf1 which are gene editing scissors, and various intracellular proteins (e.g., transcription factors), and the like.

The nucleic acid may be one or more kinds selected from the group consisting of small interfering RNA (siRNA), ribosome ribonucleic acid (rRNA), ribonucleic acid (RNA), deoxyribonucleic acid (DNA), complementary deoxyribonucleic acid (cDNA), aptamer, messenger ribonucleic acid (mRNA), transfer ribonucleic acid (tRNA), antisense oligonucleotide, shRNA, miRNA, ribozyme, PNA, DNAzyme and sgRNA for gene editing, and the like, but not limited thereto.

Herein, the term "siRNA" refers to double stranded RNA (duplex RNA) which can induce RNAi (RNA interference) through cleavage of specific mRNA, or single stranded RNA that has a double stranded form inside the single stranded RNA. It consists of a sense RNA strand having the sequence homologous to mRNA of a target gene and an antisense RNA strand having the sequence complementary thereto. As siRNA can inhibit expression of a target gene, it is provided by an effective gene knock-down method or method of gene therapy. Binding between double strands is carried out through hydrogen bonding between nucleotides, and not all nucleotides within the double strand must be complementary and completely bound.

The length of siRNA may be about 15 to 60, specifically about 15 to 50, about 15 to 40, about 15 to 30, about 15 to 25, about 16 to 25, about 19 to 25, about 20 to 25, or about 20 to 23 nucleotides. The siRNA length means the number of nucleotides on one side of the double stranded RNA, that is, the number of base pairs, and in case of single stranded RNA, means the length of the double strand inside the single stranded RNA. In addition, siRNA may be composed of nucleotides introduced with various functional groups for the purpose of increasing blood stability or weakening an immune response, and the like.

Herein, the term "antisense oligonucleotide" may be modified at a position of one or more bases, sugars or backbones to enhance the efficacy (De Mesmaeker et al., Curr Opin Struct Biol., 5(3):343-55, 1995). The oligonucleotide backbone may be modified by phosphorothioate, phosphotriester, methyl phosphonate, short-chain alkyl, cycloalkyl, short-chain heteroatomic, heterocyclic intersaccharide binding, and the like. In addition, the antisense oligonucleotide may comprise one or more substituted sugar moieties. The antisense oligonucleotide may comprise a modified base. The modified base includes hypoxanthine, 6-methyladenine, 5-me pyrimidine (particularly, 5-methylcytosine), 5-hydroxymethylcytosine (HMC), glycosyl HMC, gentiobiosyl HMC, 2-aminoadenne, 2-thiouracil, 2-thiothymine, 5-bromouracil, 5-hydroxymethyluracil, 8-azaguanine, 7-deazaguanine, N6(6-aminohexyl)adenine, 2,6-diaminopurine, and the like.

Herein, "single stranded deoxyribonucleic acid (ssDNA)" means a single stranded oligonucleotide which binds

to specific target DNA selectively and induces an antigene effect.

Herein, "aptamer" means an oligonucleotide (generally, 20 ~ 80 nt DNA or RNA) which binds to a specific target. Preferably, herein, "aptamer" means an oligonucleotide aptamer (e.g., DNA or RNA aptamer).

Herein, "mRNA" means synthetic mRNA (in vitro transcribed mRNA) capable of expressing a gene.

Herein, "shRNA" means single-stranded 50 to 70 nucleotides, and forms a stem-loop (stemloop) structure in vivo. On both sides of the loop of 5 to 10 nucleotides, complementarily, long RNA of 19 to 29 nucleotides are base-paired to form a double-stranded stem.

Herein, "miRNA (microRNA)" means a single stranded RNA molecule which controls gene expression and consists of full length 21 to 23 nucleotides. miRNA is an oligonucleotide which is not expressed in a cell, and has a short stem-loop structure. miRNA has full or partial homology with one or two or more mRNA (messenger RNA) and suppresses target gene expression through complementary binding to the mRNA.

Herein, "ribozyme" is a kind of RNA, and is RNA which recognizes a nucleotide sequence of specific RNA and has the same function as enzyme cutting it by itself. The ribozyme is a complementary nucleotide sequence of a target messenger RNA strand and consists of a region that binds with specificity and a region that cuts the target RNA.

Herein, "DNAzyme" is a single stranded DNA molecule having enzyme activity, and DNAzyme consisting of 10 to 23 nucleotides (10-23 DNAzyme) cuts a RNA strand at a specific position under the physiologically similar condition. The 10-23 DNAzyme cuts between any exposed purines and pyrimidines without base pairing. The 10-23 DNAzyme consists of an active site (catalytic domain) of enzyme consisting of 15 conserved nucleotide sequences (for example, 5'-GGCTAGCTACAACGA-3') and an RNA substrate binding domain consisting of 7 ~ 8 DNA nucleotide sequences which recognize RNA substrates to the left and right of the active domain of the enzyme afore-described.

Herein, "PNA (Peptide nucleic acid)" is a molecule having all properties of nucleic acids and proteins, and means a molecule capable of complementarily binding to DNA or RNA. The PNA was first reported in 1999 as similar DNA in which nucleobases are linked by peptide bonds (document [Nielsen PE, Egholm M, Berg RH, Buchardt O, "Sequence-selective recognition of DNA by strand displacement with a thymine-substituted polyamide", Science 1991, Vol. 254: pp1497-1500]). The PNA is not found in the natural world and is artificially synthesized by a chemical method. The PNA causes a hybridization reaction with a natural nucleic acid of a complementary nucleotide sequence to form a double strand. PNA/DNA double strands are more stable than DNA/DNA double strands for the same length. As a backbone of peptides, N-(2-aminoethyl)glycine repeatedly linked by amide bonds is most commonly used, and in this case, the backbone of the peptide nucleic acid is electrically neutral different from the backbone of the natural nucleic acid. 4 nucleotides present in PNA have almost the same spatial size and distance between nucleotides as in case of the natural nucleic acid. The PNA is not only chemically more stable than the natural nucleic acid, but also biologically stable because it is not degraded by nuclease or protease.

Herein, "sgRNA" is an oligonucleotide (generally, RNA molecule) binding to a specific DNA target, and means a complex single RNA molecule of crispr RNA (crRNA) and tracer (tracrRNA). It is an RNA molecule which is used for recognizing a specific DNA sequence with Cas9 nuclease in the CRISPR system and enables selective gene cleavage, and approximately, comprises a 20-nt sequence capable of complementarily binding to DNA, and has a total length of 100 nt.

Herein, "gene editing protein" refers to Cas9, spCas9, cjCas9, casX, CasY and Cpf1, and the like, and refers to a protein that recognizes a target DNA nucleotide sequence with sgRNA to cause DNA cleavage.

The target cell to which a drug and/or nucleic acid is delivered by the lipid nanoparticle according to one embodiment may be LSECs in vivo or LSECs isolated from vivo. The composition for drug delivery and/or complex of the drug (anionic drug, nucleic acid or combination thereof) and lipid nanoparticle according to one embodiment may target or specifically target LSECs. Accordingly, the lipid nanoparticle or composition for drug or nucleic acid delivery comprising the lipid nanoparticle according to one embodiment may be for treatment of acute or chronic liver diseases such as hepatic fibrosis, liver cirrhosis, hepatitis (e.g., hepatitis A, hepatitis B, hepatitis C, etc.), and in addition, it may be utilized as a composition for delivery of a therapeutic agent (drug) absorbed through liver.

Other aspect provides a pharmaceutical composition for preventing or treating liver disease, comprising (1) the lipid nanoparticle; and (2) an anionic drug, nucleic acid or combination thereof.

The lipid nanoparticle comprised in the pharmaceutical composition for preventing or treating liver disease is same as the lipid nanoparticle comprised in the composition for drug delivery described above.

The anionic drug and nucleic acid comprised in the pharmaceutical composition for preventing or treating liver disease is same as the anionic drug and nucleic acid comprised in the composition for drug delivery described above.

The pharmaceutical composition for preventing or treating liver disease according to one embodiment may comprise a lipid nanoparticle in which an anionic drug and/or nucleic acid is encapsulated.

The liver disease may be one or more kinds selected from the group consisting of hepatitis B virus infection, acute liver failure, cirrhosis, liver fibrosis, hemophilia A, hemorrhagic necrosis, acute liver failure, and liver regeneration.

The anionic drug may have an effect for preventing or treating liver disease.

The nucleic acid may have an effect for preventing or treating liver disease, and for example, it may be siRNA

and/or miRNA which can inhibit expression such as (1) TTR (Transthyretin) ((e.g., human TTR (protein: GenBank Accession Nos. NP_000362.1; gene: GenBank Accession Nos. NM_000371.4, etc.), mouse TTR (protein: GenBank Accession No.; NP_038725.1; gene: GenBank Accession No. NM_013697.5, etc.)), (2) PCSK9 (Proprotein convertase subtilisin/kexin type 9) ((e.g., human PCSK9 (protein: GenBank Accession Nos. NP_777596.2; gene: GenBank Accession Nos. NM_174936.4, etc.), mouse PCSK9 (protein: GenBank Accession No. NP_705793.1; gene: GenBank Accession No. NM_153565.2, etc.)), (3) HBV (Hepatitis B Virus) (e.g., HBV genotype A (e.g., GenBank Accession No.: X02763, X51970, or AF090842); HBV genotype B (e.g., GenBank Accession No.: D00329, AF100309, orAB033554); HBV genotype C (e.g., GenBank Accession No.: X04615, M12906, AB014381, AB042285, AB042284, AB042283, AB042282, AB026815, AB026814, AB026813, AB026812, or AB026811); HBV genotype D (e.g., GenBank Accession No.: X65259, M32138, or X85254); HBV genotype E (e.g., GenBank Accession No.: X75657 or AB032431); HBV genotype F (e.g., GenBank Accession No.: X69798, AB036910, or AF223965); HBV genotype G (e.g., GenBank Accession No.: AF160501, AB064310, orAF405706) HBV genotype H (e.g., AY090454, AY090457, or AY090460) (4) Bax (BCL2 associated X)) ((e.g., human Bax (protein: GenBank Accession Nos. NP_001278357.1, NP_001278358.1, NP_001278359.1, NP_001278360.1, NP_004315.1 ; gene: GenBank Accession Nos. NM_001291428.2, NM_001291429.2, NM_001291430.1, NM_001291431.2, NM_004324.4, etc.), mouse Bax (protein: GenBank Accession No. NP_031553.1; gene: GenBank Accession No. NM_007527.3, etc.), (5) VEGF (Vascular endothelial growth factor) (e.g., VEGFA ((e.g., human VEGFA (protein: GenBank Accession Nos. NP_001020537.2, NP_001020538.2, NP_001020539.2, NP_001020540.2, NP_001020541.2; gene: GenBank Accession Nos. NM_003376.6, NM_001025366.3, NM_001025367.3, NM_001025368.3, NM_001025369.3, etc.), mouse VEGFA (protein: GenBank Accession No. NP_001020421.2, NP_001020428.2, NP_001103736.1, NP_001103737.1, NP_001103738.1; gene: GenBank Accession No. NM_001025250.3, NM_001025257.3, NM_001110266.1, NM_001110267.1, NM_001110268.1, etc.); VEGFB ((e.g., human VEGFB(protein: GenBank Accession Nos. NP_001230662.1, NP_003368.1; gene: GenBank Accession Nos. NM_003377.5, NM_001243733.2, etc.), mouse VEGFB (protein: GenBank Accession No. NP_001172093.1, NP_035827.1; gene: GenBank Accession No. NM_001185164.1, NM_011697.3, etc.); VEGFC (e.g., human VEGFC (protein: GenBank Accession Nos. NP_005420.1; gene: GenBank Accession Nos. NM_005429.5, etc.), mouse VEGFC (protein: GenBank Accession No.; NP_033532.1; gene: GenBank Accession No. NM_009506.2, etc.)), and/or (6) PDGF (Platelet-derived growth factor) (e.g., PDGFA ((e.g., human PDGFA (protein: GenBank Accession Nos. NP_002598.4, NP_148983.1,; gene: GenBank Accession Nos. NM_002607.5, NM_033023.4, etc.), mouse PDGFA (protein: GenBank Accession No. NP_032834.1, NP_001350200.1; gene: GenBank Accession No. NM_008808.4, NM_001363271.1, etc.)); PDGFB ((e.g., human PDGFB (protein: GenBank Accession Nos. NP_002599.1, NP_148937.1; gene: GenBank Accession Nos. NM_033016.3, NM_002608.4, etc.), mouse PDGFB (protein: GenBank Accession No. NP_035187.2; gene: GenBank Accession No. NM_011057.4, etc.)); PDGFC ((e.g., human PDGFC (protein: GenBank Accession Nos. NP_057289.1; gene: GenBank Accession Nos. NM_016205.3, etc.), mouse PDGFC (protein: GenBank Accession No. NP_064355.1, NP_001344675.1; gene: GenBank Accession No. NM_019971.3, NM_001357746.1, etc.)); PDGFD (e.g., human PDGFD(protein: GenBank Accession Nos. NP_079484.1, NP_149126.1; gene: GenBank Accession Nos. NM_033135.4, NM_025208.5, etc.), mouse PDGFD (protein: GenBank Accession No. NP_082200.1, NP_001344326.1, NP_001344327.1; gene: GenBank Accession No. NM_027924.3, NM_001357397.1, NM_001357398.1, etc.)).

The pharmaceutical composition may be administered by various routes including parenteral administration into mammals including humans, and parenteral administration may be applied intravenously, subcutaneously, intraperitoneally or locally, and the dosage varies depending on the condition and body weight of the patient, degree of disease, drug form, administration route and time, but may be appropriately selected by those skilled in the art.

When the pharmaceutical composition according to one embodiment is formulated, it is prepared by using a diluent or excipient such as a filler, an extender, a binding agent, a wetting agent, a disintegrant, a surfactant, and the like used commonly.

The formulation for parenteral administration includes a sterilized aqueous solution, a non-aqueous solvent, a suspended solvent, emulsion, a lyophilized formulation, a suppository, and the like.

As the non-aqueous solvent and suspended solvent, propylene glycol, polyethylene glycol, plant oil such as olive oil, injectable ester such as ethyl oleate, and the like may be used. As a base compound of the suppository, witepsol, macrogol, tween 61, cacao butter, laurin butter, glycerol, gelatin, and the like.

The pharmaceutical composition according to one embodiment is administered in a pharmaceutically effective dose. Herein, "pharmaceutically effective dose" means an amount sufficient to treat disease at a reasonable benefit/risk ratio applicable to medical treatment, and the effective dose level may be determined depending on factors including the patient's disease type, severity, drug activity, drug sensitivity, administration time, administration route and excretion rate, treatment period and concomitant drugs, and other factors well known in the medical field. The pharmaceutical composition according to one example may be administered as an individual therapeutic agent or may be administered in combination with other therapeutic agents, and may be administered sequentially or simultaneously with conventional therapeutic agents, and may be singly or multiply administered. It is important to administer an amount capable of obtaining the maximum effect with a minimum amount without side effects, in consideration of all of the above factors,

and this may be easily determined by those skilled in the art.

Specifically, the effective dose of the compound according to the present invention may vary depending on the patient's age, gender and body weight, and may be administered daily or every other day, or administered by dividing into 1 to 3 times a day. However, it may be increased or reduced depending on the administration route, severity of obesity, gender, body weight, age, and the like, and therefore, the above dose does not limit the scope of the present invention in any way.

In one specific example, the pharmaceutical composition may be administered in a dose of 0.1 to 100 mg/kg, 0.1 to 50 mg/kg, 1 to 10 mg/kg, or 1 to 5 mg/kg, based on the concentration of the drug (anionic drug, nucleic acid or combination thereof) comprised in the pharmaceutical composition.

[ADVANTAGEOUS EFFECTS]

According to one embodiment, the lipid nanoparticle is liver tissue-specific and/or LSECs, have excellent biocompatibility and can deliver a gene therapeutic agent with high efficiency, and thus it can be usefully used in related technical fields such as lipid nanoparticle mediated gene therapy.

[BRIEF DESCRIPTION OF THE DRAWINGS]

FIG. 1 shows an exemplary structure of the lipid nanoparticle comprising a mannose according to one embodiment.

FIG. 2 shows the result of 1H NMR (room temperature, 400MHz) of 246-C10 in $CDCl_3$.

FIG. 3a (241-C10 LNP to 243-C10 LNP) and FIG. 3b (244-C10 LNP to 246-C10 LNP) show the result of measuring the fluorescence intensity shown by each lipid nanoparticle in a solution having a range of pH 4.1 to pH 9.6.

FIG. 4a and FIG. 4b are results showing the intracellular gene delivery efficiency of each nanoparticle. Specifically, FIG. 4a shows the luminescence intensity measured by transforming LNP encapsulating mRNA (luc mRNA) encoding luciferase into HeLa cell and then dissolving the cell, and FIG. 4b shows the luminescence intensity measured by transforming LNP encapsulating luc mRNA to a hepatocyte and then dissolving the cell. In FIG. 4b, +ApoE refers to a group treated by ApoE3, and -ApoE refers to a group untreated by ApoE3.

FIG. 5a shows in vivo drug delivery distribution in a mouse to which 244-C10 LNP to 246-C10 LNP with Luc mRNA encapsulated is administered, and FIG. 5b shows drug delivery distribution to each organ of the mouse removed from the mouse in which 246-C10 LNP with Luc mRNA encapsulated is administered.

FIG. 6a shows the in vivo drug delivery distribution in the mouse in which 246-C10 LNP comprising lipid-PEG in an amount of 1.0 to 2.5 mol% is administered, and FIG. 6b shows the drug delivery and efficiency of the nanoparticle and the size of the nanoparticle, depending on the content of lipid-PEG comprised in the lipid nanoparticle.

FIG. 7 shows the result of confirming the hepatocyte targeting possibility according to the concentration of siFVII administered as encapsulated in the lipid nanoparticle through the expression of FVII.

FIG. 8 shows the size of the lipid nanoparticle and PDI value of the lipid nanoparticle according to the content of the lipid-PEG comprised in the lipid nanoparticle (left table), and shows the result of confirming the in vivo drug delivery efficiency to the hepatocyte through the expression of FVII (right graph).

FIG. 9 shows the size of the lipid nanoparticle and PDI value of the lipid nanoparticle according to the content of the lipid-PEG comprised in the lipid nanoparticle (left table), and shows the result of confirming the in vivo drug delivery efficiency to the LSEC through the expression of FVIII (right graph).

FIG. 10 shows the intracellular gene delivery efficiency of each nanoparticle according to the lipid-PEG content. 246-C10 LNP encapsulated with siFLuc comprising lipid-PEG at 1.5 to 5.0 mol% was treated to HeLa-Luc expressing Luciferase, and the drug delivery efficiency was confirmed through reduced luminescence.

FIG. 11 shows the excellent drug delivery efficiency of the nanoparticle comprising mannose-PEG-lipid conjugate through the amount of luc mRNA expression, compared to lipid nanoparticle comprising only PEG-lipid conjugate.

FIG. 12 shows the result of the migration of the nanoparticle into mannose-dependent HepG2 cells through the exprssion of luc mRNA. FIG. 12a shows the reduced intracellur migration of LNP comprising mannose-PEG in the HepG2 cells treated with CD206 receptor antibodies.

FIG. 12b shows the unreduced intracellur migration of LNP comprising galactose (manos isomer) PEG in the HepG2 cells treated with CD206 receptor antibodies.

FIG. 13a shows the selective migration of the nanoparticles encapsulated with cre mRNA comprising mannose-PEG-lipid conjugate into the liver in the LSL-tdTomato mouse model through the expression of Tomato fluorescence.

FIG. 13b shows the LSEC-specific delivery of the nanoparticles encapsulated with cre mRNA comprising mannose-PEG-lipid conjugate in the LSL-tdTomato mouse model through the expression of Tomato fluorescence along hepatic vessels.

FIG. 13c shows the superior transfection efficiency in LSEC of the nanoparticles comprising mannose-PEG-lipid conjugate compared to LNPs comprising only cre mRNA-encapsulated lipid-PEG conjugate.

FIG. 14 shows the results of confirming the LSEC target possibility of siFVIII which is encapsulated and administered in the lipid nanoparticle comprising mannose-PEG-lipid conjugate, through the expression level of FVIII.

FIG. 15 shows the results of confirming the non-migration to a hepatocyte of siFVII which is encapsulated and administered in the lipid nanoparticle comprising mannose-PEG-lipid conjugate, through the expression level of FVII.

FIG. 16 shows the LSEC target possibility according to the concentration of siFIII which is encapsulated and administered in the lipid nanoparticles comprising mannose-PEG-lipid conjugate, through the expression level of FVIII compared to the lipid nanoparticle comprising only lipid-PEG conjugate.

[MODE FOR INVENTION]

The present invention will be described in more detail by the following examples, but the scope is not intended to be limited by the following examples.

## Example 1. Preparation of ionizable lipids

### Example 1-1. Preparation of ionizable lipids

Ionizable lipids were synthesized by reacting the amine-based compounds of Table 1 below comprising a 6-membered heterocyclic tertiary amine and 1,2-epoxidodecane (hereinafter, C10) (Sigma-Aldrich, USA) at a molar ratio of 1:n (n = primary amine × 2 + secondary amine × 1).

[Table 1]

| Name | Chemical formula |
|---|---|
| 241 | 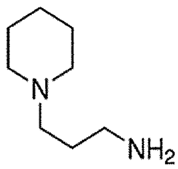 |
| 242 | 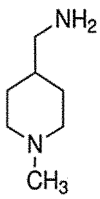 |
| 243 | 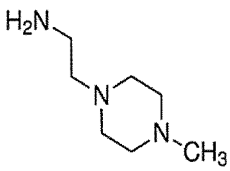 |
| 244 | 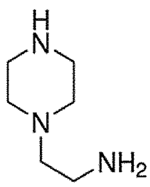 |

(continued)

| Name | Chemical formula |
|---|---|
| 245 | 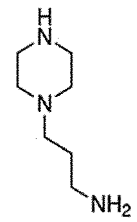 |
| 246 | 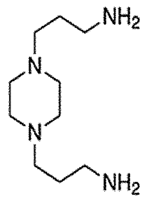 |

Specifically, each of 241 to 246 amines of the Table 1 and epoxide (C 10) were added at a molar ratio of 1:n (n = primary amine × 2 + secondary amine × 1) in a 5ml vial with a magnetic bar and reacted in a stirrer at 750rpm, 90°C for 3 days. Then, after purifying with WELUX fine silica column (Intertec, Korea), the molecular weight of each ionizable lipid produced by the reaction was calculated and they were stored at a concentration of 100mg/ml using ethanol. The ionizable lipid produced by using 241 amine and C10 was named '241-C10', and other ionizable lipids produced by using other kinds of amines were named 'used amine name (241 to 246)-C10' in the same way.

**Example 1-2. Confirmation of produced ionizable lipids**

In order to confirm the ionizable lipids produced in the Example 1-1, $^1H$ NMR was performed. Specifically, the ionizable lipid (246-C10) synthesized in Example 1-1 of 5ug was prepared by diluting in $CDCl_3$ (sigma, USA) 0.5ml to 100 mmole concentration. Then, 0.5ml each was put into a tube for 400MHz NMR and the top was sealed, and then sealed with parafilm to obtain NMR spectra using Agilent 400MHZ FT-NMR (Agilent, USA), and the result was shown in FIG. 2. As shown in FIG. 2, it could be seen that the signal representing each functional group of 246-C10 was saturated.

In addition, in order to confirm the ionizable lipids (241-C10 to 246-C10) prepared in Example 1-1, MS analysis was performed. Specifically, the ionizable lipids were diluted in ethanol at a concentration of 0.5ppm or less and MS analysis was performed. The equipment used for the analysis was 6230 LC/MS of Agilent Technologies (Palo Alto, USA) and the Zorbax SB-C18 (100 mmX2.1 mm i.d., 3.5 μm) of Agilent Technologies was used for the separation tube, and two solvents of distilled water (A) containing 0.1% formic acid and acetonitrile (B) were gradient eluted. The solvent gradient of the mobile phase was maintained for 4 minutes until the ratio of the organic solvent acetonitrile (B) was initially increased from 30% to 80% for 2 minutes and then the ratio of the organic solvent was lowered to 30% again and stabilized. The flow rate of the mobile phase was 300$\mu\ell$/min, and then, the injection volume of the analyzer was 2$\mu\ell$. The result of performing the MS analysis was shown in Table 2 below. As shown in Table 2, it could be confirmed that the measured m/z ratio and calculated m/z ratio of the ionizable lipids were almost identical.

[Table 2]

| | Chemical formula | Calculated m/z ratio | Observed m/z ratio |
|---|---|---|---|
| 241-C10 | $C_{32}H_{66}N_2O_2$ | 510.87864 | 511.5201 |
| 242-C10 | $C_{31}H_{64}N_2O_2$ | 496.85206 | 497.5043 |
| 243-C10 | $C_{31}H_{65}N_3O_2$ | 511.8667 | 513.5186 |
| 244-C10 | $C_{42}H_{87}N_3O_3$ | 682.15848 | 682.6821 |
| 245-C10 | $C_{43}H_{89}N_3O_3$ | 696.18506 | 696.7045 |
| 246-C10 | $C_{58}H_{120}N_4O_4$ | 937.5978 | 937.9383 |

From the result, it could be confirmed that the ionizable lipids were well made in Example 1-1.

**Example 2. Preparation of lipid nanoparticles**

**Example 2-1. Preparation of lipid nanoparticles**

The ionizable lipids (241-C10 to 246-C10) prepared in the Example 1-1, cholesterol (Cholesterol powder, BioReagent, suitable for cell culture, ≥99%, sigma, Korea), phospholipid (DSPC) (Avanti, US), and a lipid-PEG conjugate (ceramide-PEG conjugate; C16 PEG2000 Ceramide, Avanti, US) were dissolved in ethanol at a molar ratio of 42.5:13:43:1.5.

The ethanol in which the ionizable lipids, cholesterol, phospholipid and lipid-PEG were dissolved and acetate buffer were mixed with a microfluid mixing device (Benchtop Nanoassemblr; PNI, Canada) at a flow rate of 12ml/min in a volume ratio of 1:3, thereby preparing lipid nanoparticles (LNPs).

**Example 2-2. Preparation of nucleic acid-encapsulated lipid nanoparticles**

The ionizable lipids (241-C10 to 246-C10) prepared in the Example 1-1, cholesterol (Cholesterol powder, BioReagent, suitable for cell culture, ≥99%, sigma, Korea), phospholipid (DSPC or DOPE) (18:0 PC (DSPC), 18:1 (Δ9-Cis) PE (DOPE), Avanti, US), and a lipid-PEG conjugate (ceramide-PEG conjugate; C16 PEG2000 Ceramid, Avanti, US) were dissolved in ethanol. An RNA therapeutic agent, mRNA (luciferase mRNA; SEQ ID NO: 1) 30$\mu$g was diluted in sodium citrate 0.75ml, or siRNA (siLUC, siGFP, Anti-HPV16 E6/E7 siRNA, Anti-PMVK siRNA etc.) 30$\mu$g was diluted in sodium acetate(50mM) 0.75ml to prepare an aqueous phase.

The aqueous phase (sodium acetate or sodium citrate) in which the organic phase (ethanol) in which the ionizable lipids, cholesterol, phospholipid and lipid-PEG conjugate (hereinafter, lipid-PEG) were dissolved and an RNA therapeutic agent (nucleic acid) were dissolved were mixed through a microfluid mixing device (Benchtop Nanoassemblr; PNI, Canada) at a flow rate of 12 mℓ/min, to prepare lipid nanoparticles (LNPs) in which the nucleic acid was encapsulated. (i) In order to prepare a lipid nanoparticle in which mRNA is encapsulated, the ionizable lipid : phospholipid (DOPE): cholesterol : lipid-PEG (C16-PEG2000 ceramide) were dissolved in ethanol at a molar ratio of 26.5 : 20 : 52.5 to 51 : 1.0 to 2.5 (adjusting the content of cholesterol and lipid-PEG so that the total sum of the molar ratio is 100), and the organic phase and the aqueous phase were mixed so that the mRNA (luciferase mRNA; SEQ ID NO: 1) : ionizable lipid was at the weight ratio of 1:10, and thereby a lipid nanoparticle was prepared. (ii) In order to prepare a lipid nanoparticle in which siRNA is encapsulated, the ionizable lipid : phospholipid (DSPC): cholesterol : lipid-PEG (C16-PEG2000 ceramide) were dissolved in ethanol at a molar ratio of 42.5 : 13 : 44 to 39.5 : 0.5 to 5.0 (adjusting the content of cholesterol and lipid-PEG so that the total sum of the molar ratio is 100), and the organic phase and the aqueous phase were mixed so that the siRNA (siFVII; SEQ ID NOs: 2 and 3 were mixed at the same molar ratio, or siFVIII; SEQ ID NOs: 4 to 11 were mixed at the same molar ratio, or siLuc: SEQ ID NOs: 12 and 13 were mixed at the same ratio): ionizable lipid was at the weight ratio of 1:7.5 and thereby a lipid nanoparticle (LNP) was prepared.

The used siRNA sequences are as follows:

SEQ ID NO: 2 (FVII target siRNA sense; 5'-GGAUCAUCUCAAGUCUUACdtdt-3'), SEQ ID NO: 3 (FVII target siRNA antisense; 5'-GUAAGACUUGAGAUGAUCCdtdt-3'), SEQ ID NO: 4 (FVIII target siRNA_sense_1; 5'CUUAUAUCGUGGAGAAUUAdtdt-3') SEQ ID NO: 5 (FVIII target siRNA_antisense_1; 5'-UAAUUCUCCACGAUAUAAGdtdt-3'), SEQ ID NO: 6 (FVIII target siRNA _sense _2; 5'-UCAAAGGAUUCGAUGGUAUdtdt-3'), SEQ ID NO: 7 (FVIII target siRNA_antisense_2; 5'-AUACCAUCGAAUCCUUUGAdtdt-3'), SEQ ID NO: 8(FVIII target siRNA_sense_3; 5'-CAAGAGCACUAGUGAUUAUdtdt-3'), SEQ ID NO: 9(FVIII target siRNA_antisense_3; 5'-AUAAUCACUAGUGCUCUUGdtdt-3'), SEQ ID NO: 10(FVIII target siRNA_sense 4; 5'-GGGCACCACUCCUGAAAUAdtdt-3'), SEQ ID NO: 11(FVIII target siRNA _antisense 4; 5'-UAUUUCAGGAGUGGUGCCCdtdt-3'), SEQ ID NO: 13(siFLuc_sense; 5'-AACGCUGGGCGUUAAUCAAdtdt-3'), SEQ ID NO: 14(siFLuc_antisense; 5'-UUGAUUAACGCCCAGCGUUdtdt-3').

The prepared LNPs were dialyzed against PBS for 16 hours using a 3,500 MWCO dialysis cassette to remove ethanol and adjust the body pH and the pH of the nanoparticles.

The lipid nanoparticles comprising the ionizable lipid '241-C10' were named '241-C10 LNP', and the lipid nanoparticles prepared by using the ionizable lipid comprising amine (including lipid nanoparticles in which a nucleic acid was encapsulated) were named 'comprised amine name (241 to 246)-C10 LNP'.

**Example 3. pKa of lipid nanoparticles**

In the present example, pKa of each lipid nanoparticle (LNP) formulated in the Example 2-1 was calculated through In vitro TNS assay. Anionic TNS becomes lipophilic by interacting with a positively charged ionizable lipid, and as the pH value becomes close to the pKa value of each LNP, the lipophilic property of TNS becomes lower and more water molecules quench the TNS fluorescence, and therefore, lipid nanoparticles having a pKa of 6.0 to 7.0 have excellent in vivo drug delivery efficiency, and lipid nanoparticles showing a "s-type curve" in the graph representing fluorescence

according to pH mean that they are easy to interact with the endosome membrane and can easily escape the endosome during acidification.

Specifically, the pH of the solution comprising 20mM sodium phosphate, 25mM citrate, 20mM ammonium acetate, and 150mM NaCl with 0.1N NaOH and/or 0.1N HCl at an interval of 0.5 from pH 4.1 to pH 9.6 to prepare solutions of various pH units. 100$\mu\ell$ of each solution having each pH (pH with an interval of 0.5 from pH 4.1 to pH 9.6) was added to a black 96well plate and each was added to a solution having the pH in the range so as to be the final concentration of 6uM using a TNS stock solution of 300uM. 241-C10 LNP to 246-C10 LNP were added to the mixed solution so that the final concentration is 20uM. The fluorescence intensity was measured by excitation at 325 nm and emission at 435 nm through a Tecan equipment, and the fluorescence intensity for each lipid nanoparticle was shown in FIG. 3a and FIG. 3b, and the pKa for each lipid nanoparticle was calculated as a pH value reaching half of the maximum fluorescence and shown in Table 4 below. As shown in FIG. 3b, it could be seen that 244-C10 LNP to 246-C10 LNP exhibit a fluorescence titration s-shaped curve through nonlinear regression.

[Table 4]

| Lipid nanoparticles | pKa |
|---|---|
| 241-C10 LNP | 7.7 |
| 242-C10 LNP | 8.7 |
| 243-C10 LNP | 8.2 |
| 244-C10 LNP | 6.8 |
| 245-C10 LNP | 6.9 |
| 246-C10 LNP | 7 |

As confirmed in the Table 4, it was confirmed that the lipid nanoparticles according to one example showed pKa 6.0 to 7.0 range in which in vivo safety and drug release are excellent.

The LNPs in which a nucleic acid was encapsulated, prepared by the method as Example 2-2, also showed the same pattern according to the type of ionizable lipids contained (type of amine contained in the ionizable lipids).

## Example 4. Confirmation of characteristics of lipid nanoparticles

### Example 4-1. Particle size measurement

In the present example, the size of the lipid nanoparticles (LNP; comprising 1.5 mol% of lipid-PEG) in which mRNA was encapsulated measured in Example 2-2 was to be measured. It was diluted using PBS so that the concentration of RNA (luciferase mRNA; SEQ ID NO: 1) comprised in each lipid nanoparticle prepared in Example 2-2 was 1$\mu$g/ml, and the diameter and polydispersity index (PDI) of the LNPs were measured using dynamic light scattering (DLS) in Malvern Zetasizer Nano (Malvern Instruments, UK), and the result was described in Table 5 below.

[Table 5]

| Lipid nanoparticles | Diameter (nm) | PDI |
|---|---|---|
| 241-C10 LNP | 128 | 0.259 |
| 242-C10 LNP | 77 | 0.210 |
| 243-C10 LNP | 56 | 0.225 |
| 244-C10 LNP | 66 | 0.149 |
| 245-C10 LNP | 70 | 0.210 |
| 246-C10 LNP | 68 | 0.143 |

As confirmed in the Table 5, the lipid nanoparticles according to one example showed the particle size that is easy to be introduced into hepatocytes and has excellent drug release, and it could be found that the PDI values were small and the particles were uniform in order of 241-C10 LNP > 243-C10 LNP > 242-C10 LNP = 245-C10 LNP > 244-C10 LNP > 246-C10 LNP.

**Example 4-2. Measurement of encapsulation efficiency**

The encapsulation efficiency (drug encapsulation efficiency, %) of each LNP (comprising 1.5 mol% of lipid-PEG) in which siRNA (siFVII siRNA) was encapsulated as a nucleic acid drug was measured through Ribogreen analysis (Quant-iT™ RiboGreen® RNA, Invitrogen). The LNPs in which a nucleic acid drug was encapsulated prepared in the Example 2-2 were diluted with 1xTE buffer solution 50$\mu\ell$ in a 96 well plate so that the final concentration of siRNA was 4 ~ 7 $\mu$g/ml. To the group untreated with Triton-X (Triton-x LNP(-)), 1xTE buffer 50$\mu\ell$ was added, and to the group treated with Triton-X (Triton-x LNP(+)), 2% Triton-X buffer 50$\mu\ell$ was added. By incubating at 37°C for 10 minutes, the nucleic acid encapsulated by degrading LNPs with Triton-X was released. Then, Ribogreen reagent 100$\mu\ell$ was added to each well. The fluorescence intensity (FL) of Triton LNP(-) and Triton LNP(+) was measured by the wavelength bandwidth (excitation : 485nm, emission : 528nm) in Infinite® 200 PRO NanoQuant (Tecan), and the drug encapsulation efficiency (encapsulation efficiency, %) was calculated as the following Equation 3. The drug encapsulation efficiency (%) for each LNP was shown in Table 6 below as the average value of the results measured repeatedly twice.

(Equation 3)

Drug encapsulation efficiency (%)=(Fluorescence intensity of Triton LNP(+) – Fluorescence intensity of Triton LNP(-))/(Fluorescence intensity of Triton LNP(+)) X 100

[Table 6]

| Lipid nanoparticles | Encapsulation efficiency (%) |
|---|---|
| 241-C10 LNP | 84 |
| 242-C10 LNP | 83 |
| 243-C10 LNP | 91 |
| 244-C10 LNP | 87 |
| 245-C10 LNP | 91 |
| 246-C10 LNP | 94 |

As confirmed in the Table 6, it was confirmed that the lipid nanoparticles according to one example could encapsulate a drug with high efficiency.

**Example 5. Confirmation of intracellular nucleic acid delivery using lipid nanoparticles**

**Example 5-1. Nucleic acid delivery effect according to types of ionizable lipids comprised in LNP**

One day prior to transfection of LNP according to one example into cells, HeLa cells (Korea Cell Line Bank) were aliquoted at $0.01\times10^6$ cells/well in a white plate (96well) and were cultured under the condition of 37°C, 0.5~3% $CO_2$ in DMEM media (SH30022, Hyclone, USA). After stirring LNPs (241-C10 LNP to 246-C10 LNP comprising 1.5mol% of lipid-PEG) in which mRNA (luc mRNA; SEQ ID NO: 1) encoding a luciferase gene with ApoE3 0.1$\mu$g/ml by pipetting and then incubating at a room temperature for 10 minutes, they were treated (100ng/well based on the mRNA comprised in the lipid nanoparticles) in HeLa cells. ApoE3 binds to the LNP surface and plays a role in allowing LNP to enter the cell through endocytosis through an LDL receptor expressed on the cell surface.

In 24 hours, after treating 100$\mu\ell$/well of Bright-Glo™ Luciferase Assay solution (promega, USA) each and leaving them at a room temperature for 10 minutes, the luminescence intensity was measured for the dissolved cells using Infinite M200 luminescence measuring device (Tecan, USA), and the result was shown in FIG. 4a. As shown in FIG. 4a, 244-C10 LNP, 245-C10 LNP, and 246-C10 LNP having a pKa range of 6.0 to 7.0 showed strong luminescence intensity, and among them, 246-C10 LNP had the highest luminescence intensity, and therefore, it could be seen that 246-C10 LNP had the highest intracellular drug delivery efficiency.

**Example 5-2. Confirmation of nucleic acid delivery in hepatocytes**

The luminescence intensity was measured by delivering luc mRNA into hepatocytes using 246-C10 lipid nanoparticle prepared in Example 2-2, thereby confirming expression of the gene.

Specifically, after combining 246-C10 LNP (comprising 1.5 mol% of lipid-PEG) in which luc mRNA (SEQ ID NO: 1) was encapsulated with ApoE3 5ug/ml, the LNP was treated into a hepatocyte cell line (Nexel, Korea) aliquoted at 1x105cells/well at 0.2ug/well, 0.5ug/well, or 1ug/well based on the mRNA concentration comprised in the nanoparticle. In 6 hours, Bright-Glo™ Luciferase Assay solution (promega, USA) of 100$\mu\ell$/well was treated and left at a room temperature for 10 minutes, and then the luminescence intensity was measured for the dissolved cells using Infinite M200 luminescence measuring device (Tecan, US) and the result was shown in FIG. 4b.

As confirmed in FIG. 4b, it was confirmed that the lipid nanoparticle according to one example was easy to introduce into cells through binding to ApoE3, increased the amount of drug (nucleic acid) delivery in a concentration-dependent manner, and could deliver the drug to hepatocytes with high efficiency.

**Example 6. Confirmation of in vivo expression using lipid nanoparticles**

As confirmed in the Example 5-1, in vivo drug delivery efficiency and biodistribution of 244-C10 LNP to 246-C10 LNP showing an excellent gene expression effect (gene delivery effect) in vitro were to be confirmed in the present example.

244-C10 to 246-C10 LNP (comprising 1.5 mol% of lipid-PEG) in which luc mRNA (SEQ ID NO: 1) was encapsulated by the method of Example 2-2 were prepared, and each nanoparticle was dialyzed in PBS for 16 hours to remove ethanol. In 3 hours after intravenously (i.v) injecting the lipid nanoparticle in which mRNA was encapsulated into C57BL/6 Female 7-week-old mice (Orient Bio) in an amount of 0.1mg/kg based on the mRNA comprised in the lipid nanoparticle, luciferin 0.25 mg/kg was intraperitoneally administered and the bioluminescence was confirmed through IVIS (PerkinElmer, USA) equipment, and the result was shown in FIG. 5a.

Mice in which luc mRNA-encapsulated 246-C10 LNP was administered were sacrificed and organs were removed, and the biodistribution of the lipid nanoparticle was confirmed in each organ through IVIS equipment and the result was shown in FIG. 5b.

As shown in FIG. 5a, mice in which luc mRNA-encapsulated 244-C10 LNP to 246-C10 LNP were administered showed high luminescence intensity, and this corresponds to the result of the Example 5-1. In particular, as shown in FIG. 5a and FIG. 5b, through systemic imaging and ex vivo organ imaging, it was confirmed that luc mRNA-encapsulated 246-C10 LNP showed high luminescence intensity specifically to liver, and thereby it could be confirmed that the lipid nanoparticle according to one example showed high biodistribution to the liver.

**Example 7. Confirmation of composition ratio of lipid nanoparticles optimal for nucleic acid delivery**

In the present example, the composition ratio of the lipid nanoparticle with the most excellent drug delivery efficiency specifically to liver in vivo was to be confirmed.

In the preparation of the lipid nanoparticle, the lipid nanoparticle (246-C10 LNP) in which luc mRNA (SEQ ID NO: 1) was encapsulated was prepared by the method of Example 2-2 by mixing lipid-PEG (C16-PEG2000 ceramide) at1.0 to 2.5 mol%. The weight ratio of the ionizable lipid : mRNA comprised in the lipid nanoparticle was 10 : 1, and the molar ratio of the ionizable lipid (246-C10) : phospholipid (DOPE) : cholesterol : lipid-PEG (C16-PEG2000 ceramide) comprised in the LNP was 26.5 : 20 : 52.5 to 51 : 1.0 to 2.5 (adjusting the content of cholesterol and lipid-PEG so that the total sum of the molar ratio is 100).

For the 246-C10 LNP in which lipid-PEG was contained at 1.0 mol%, 1.5 mol%, or 2.5 mol% and luc mRNA was encapsulated, similarly to the method of Example 6, in 3 hours after mRNA-encapsulated lipid nanoparticle was intravenously (i.v) injected to C57BL/6 Female 7-week-old mice (Orient Bio) at a dose of 0. 1mg/kg based on the luc mRNA contained in the lipid nanoparticle, luciferin 0.25 mg/kg was intraperitoneally administered through IVIS (PerkinElmer, USA) equipment to confirm bioluminescence, and the result was shown in FIG. 6, and the size of the lipid nanoparticles according to the lipid-PEG content was measured as same as the method of Example 4-1 and was described in Table 6 and FIG. 6 below.

[Table 6]

| Lipid-PEG content comprised in LNP | Diameter (nm) |
|---|---|
| 1.0 mol% | 90 |
| 1.5 mol% | 67 |

(continued)

| Lipid-PEG content comprised in LNP | Diameter (nm) |
|---|---|
| 2.5 mol% | 55 |

As shown in FIG. 6a and FIG. 6b, it could be confirmed that the group in which the lipid nanoparticle according to one example was administered had excellent drug delivery efficiency to the liver, and the LNP size comprising lipid-PEG of 1.5 mol% was about 70nm.

## Example 8. Confirmation of hepatocyte-specific drug delivery effect

### Example 8-1. Confirmation of knockout effect of FVII using lipid nanoparticles

FVII is expressed specifically in hepatocytes and therefore, in the present example, the hepatocyte targetability of the lipid nanoparticles according to one example was to be confirmed through an FVII (Factor VII) knockout effect using siFVII.

So that a concentration based on the concentration of siRNA comprised in the lipid nanoparticle was 0.03mg/kg, 0. 1mg/kg, or 0.3mg/kg, in 3 days after the 246-C10 lipid nanoparticle (comprising lipid-PEG of 1.5 mol%) in which FVII target siRNA (SEQ ID NOs: 2 and 3) was encapsulated, prepared in Example 2-2 was intravenously injected to C57BL/6 female 7-week-old 20g mice, blood was collected through tail veins, and blood analysis was performed according to the protocol of the coaset FVII assay kit, and a standard curve was drawn with blood of mice administered with PBS and the FVII expression was measured and the result was shown in FIG. 7. As shown in FIG. 7, as FVII expression was inhibited in vivo dependently on the siRNA concentration encapsulated in the 246-C10 lipid nanoparticle, it was confirmed that the lipid nanoparticle according to one example could deliver a nucleic acid to hepatocytes as a target.

### Example 8-2. Drug delivery effect to hepatocytes according to lipid-PEG content

The lipid nanoparticle (246-C10 LNP) in which siFVII (SEQ ID Nos: 2 and 3) was encapsulated by the method of Example 2-2, by modifying the content of lipid-PEG comprised in the lipid nanoparticle to 0.5 to 5.0 mol%, was prepared. The weight ratio of the ionizable lipid : siRNA comprised in the lipid nanoparticle was 7.5 : 1, and the molar ratio of the ionizable lipid (246-C10) : phospholipid (DSPC) : cholesterol : lipid-PEG (C16-PEG2000 ceramide) comprised in the LNP was 42.5 :13 : 44 to 39.5 : 0.5 to 5.0 (adjusting the content of cholesterol and lipid-PEG so that the total sum of the molar ratio is 100).

The diameter and polydispersity index of the lipid nanoparticles prepared above were measured as same as the method of Example 4-1, and were shown in Table 7 and FIG. 8 (left table) below.

[Table 7]

| **Lipid-PEG (%)** | **Average diameter (nm)** | **PDI** |
|---|---|---|
| 0.5 | 120 | 0.018 |
| 1 | 78 | 0.106 |
| 1.5 | 52 | 0.159 |
| 3 | 42 | 0.152 |
| 5 | 37 | 0.226 |

So that a concentration based on the concentration of siRNA comprised in the lipid nanoparticle was 0.2mg/kg, in 3 days after the lipid nanoparticle (comprising lipid-PEG of 0.5 to 5 mol%) in which siFVII was encapsulated was intravenously injected to C57BL/6 female 7-week-old 20g mice, blood was collected through tail veins, and similarly to the method of Example 8-1, using coaset FVII assay kit, the FVII expression was measured and the result was shown in FIG. 8 (right graph). As shown in FIG. 8, it was confirmed that when the lipid nanoparticle according to one example was administered, the FVII expression in vivo was reduced, and when the lipid nanoparticle having a lipid-PEG content of 0.5 to 5.0 mol%, or 0.5 to 3.0 mol% was administered, the FVII expression was excellently inhibited.

**Example 9. LSEC-specific drug delivery effect**

As FVIII is specifically expressed in LSEC (liver sinusoidal endothelial cells), in the present example, the LSEC targetability of the lipid nanoparticle according to one example was to be confirmed through a knockout effect of FVIII (Factor VIII) using siFVIII, and the drug delivery effect according to the lipid-PEG content was examined.

By modifying the content of lipid-PEG comprised in the lipid nanoparticle to 0.5 to 5.0 mol%, lipid nanoparticles (246-C10 LNP) in which siFVIII (SEQ ID Nos: 4 to 11) was encapsulated were prepared by the method of Example 2. The weight ratio of the ionizable lipid : siRNA comprised in the lipid nanoparticle was 7.5 : 1, and the ionizable lipid (246-C10) : phospholipid (DSPC) : cholesterol : lipid-PEG (C16-PEG2000 ceramide) comprised in the LNP = 42.5 : 13 : 44 to 39.5 : 0.5 to 5.0 (adjusting the content of cholesterol and lipid-PEG so that the total sum of the molar ratio is 100).

The diameter and PDI of the lipid nanoparticles prepared above were measured by the same method of Example 4-1, and were shown in Table 8 and FIG. 9 (left table) below.

[Table 8]

| Lipid-PEG (%) | Average diameter (nm) | PDI |
|---|---|---|
| 0.5 | 166 | 0.018 |
| 1 | 87 | 0.106 |
| 1.5 | 78 | 0.159 |
| 3 | 42 | 0.152 |
| 5 | 35.6 | 0.226 |

So that a concentration based on the concentration of siRNA comprised in the lipid nanoparticle was 0.5mg/kg, in 2 days after the lipid nanoparticle (comprising lipid-PEG of 0.5 to 5 mol%) in which siFVIII was encapsulated was intravenously injected to C57BL/6 female 7-week-old 20g mice, blood was collected through tail veins, and similarly to the method of Example 8-1, using coaset FVII assay kit, the FVIII expression was measured and the result was shown in FIG. 9 (right graph). As shown in FIG. 9, it was confirmed that when the lipid nanoparticle according to one example was administered, the FVIII expression in vivo was reduced, and the lipid nanoparticle according to one example could target the LSEC, and when the lipid nanoparticle having a lipid-PEG content of 0.5 to 5.0 mol%, or 0.5 to 3.0 mol% was administered, the FVIII expression was excellently inhibited.

**Example 10. Preparation of lipid nanoparticles comprising mannose**

**Example 10-1. Preparation of lipid nanoparticles comprising mannose**

The ionizable lipid (246-C10) prepared in the Example 1-1 above, cholesterol (Cholesterol powder, BioReagent, suitable for cell culture, >_99%, sigma, Korea), phospholipid (DSPC or DOPE) (Avanti, USA), lipid-PEG conjugate (C16-PEG2000 ceramide, or ceramide-PEG conjugate; C16 PEG2000 Ceramide, Avanti, USA) and mannose-PEG-lipid conjugate (mannose-PEG-DSPE conjugate; Biochempeg, USA) were dissolved in ethanol at a molar ratio of 42. 5: 13:43 to 41.5:0.5: 1.0 to 2.5 (adjustment of cholesterol and total lipid-PEG content (= ceramide-PEG conjugate content + mannose-PEG-DSPE conjugate content) so that the total sum of the molar ratios was 100). The ethanol and acetate buffers containing ionizable lipids, cholesterol, phospholipid, ceramide-PEG conjugate, and mannose-PEG-lipid conjugate (mannose-PEG-DSPE) were mixed using a microfluidic mixing device (Benchtop Nanoassemblr; PNI, Canada) at a flow rate of 12 ml/min in a volume ratio of 1:3 to prepare lipid nanoparticles comprising mannose-PEG-lipid conjugate (hereinafter, referred to as mannose-LNPs).

**Example 10-2. Preparation of nucleic acid-encapsulated mannose-comprising lipid nanoparticles**

The ionizable lipid (246-C10) prepared in Example 1-1 above, cholesterol (Cholesterol powder, BioReagent, suitable for cell culture, >_99%, sigma, Korea), phospholipid (DSPC or DOPE) (18:0 PC (DSPC), 18: 1 (Δ9-Cis) PE (DOPE), Avanti, USA), lipid-PEG conjugate (C16-PEG2000 Ceramid, Avanti, USA), and mannose-PEG-lipid conjugate (mannose-PEG-DSPE conjugate, Biochempeg, USA) were dissolved in ethanol. The aqueous phase was prepared by diluting 30 μg of mRNA (luciferase mRNA; SEQ ID NO: 1 or Cre; SEQ ID NO: 12) in 0.75 ml of sodium citrate, or 30 μg of siRNA (siFVII; SEQ ID NOs: 2 and 3, or siFVIII; SEQ ID NOs: 4 to 11, or siFluc; SEQ ID NOs: 13 and 14) in 0.75 ml of 50 mM sodium acetate.

The organic phase (ethanol) comprising ionizable lipids, cholesterol, phospholipid, lipid-PEG conjugate (C16-PEG2000 ceramide), and mannose-PEG-lipid conjugate (mannose-PEG-DSPE conjugates) and the aqueous phase (sodium acetate or sodium citrate) comprising nucleic acids (the above mRNAs or siRNAs) were mixed at a flow rate of 12 ml/min using a microfluidic mixing device (Benchtop Nanoassemblr; PNI, Canada) to prepare nucleic acid-encapsulated mannose-comprising lipid nanoparticles (mannose-LNPs). (i) In order to prepare a lipid nanoparticle in which mRNA is encapsulated, the ionizable lipid (246-C10) : phospholipid (DOPE) : cholesterol : C16-PEG2000 ceramide (ceramide-PEG conjugate) : mannose-PEG-lipid conjugate (mannose-PEG-DSPE conjugate) was mixed in a molar ratio of 26.5 : 20 : 50.5 to 52 : 0.5 : 1.0 to 2. 5 (cholesterol and total lipid-PEG content (= ceramide-PEG conjugate content + mannose-PEG-DSPE conjugate content) adjusted so that the total sum of the molar ratios was 100) were dissolved in ethanol, and lipid nanoparticles were prepared by mixing the organic phase and the aqueous phase so that the weight ratio of mRNA (luciferase mRNA; SEQ ID NO: 1 or Cre; SEQ ID NO: 12) : ionizable lipid was 1:10. (ii) In order to prepare a lipid nanoparticle in which siRNA is encapsulated, the ionizable lipid : phospholipid (DSPC) : cholesterol : C16-PEG2000 ceramide : mannose-PEG-DSPE conjugate were dissolved in ethanol at a molar ratio of 42. 5 : 13 : 43 to 41.5 : 0.5 : 1.0 to 2. 5 (the content of cholesterol and total lipid-PEG (= ceramide-PEG conjugate content + mannose-PEG-DSPE conjugate content) were adjusted so that the total sum of the molar ratio is 100), and lipid nanoparticles were prepared by mixing the organic and aqueous phases in a weight ratio of siRNA (siFVII; SEQ ID NOs: 2 and 3, siFVIII; SEQ ID NOs: 4 to 11, or siFluc; SEQ ID NOs: 13 and 14) : ionizable lipid of 1 : 7.5. FIG. 1 shows an exemplary structure of a lipid nanoparticle comprising mannose, in which a nucleic acid is embedded, according to one embodiment.

The prepared LNPs were dialyzed against PBS for 16 hours using a 3500 MWCO dialysis cassette to remove ethanol and adjust the pH of the lipid nanoparticles with the pH of the body.

Hereinafter, the lipid nanoparticle comprising mannose-PEG-lipid conjugate were named 'mannose-LNP' .

**Example 11. ApoE adsorption rate and intracellular migration according to lipid-PEG conjugate content**

**Example 11-1. Measurement of ApoE adsorption rate**

As in the method of Example 2 above, in order to prepare lipid nanoparticles encapsulated with mRNA (SEQ ID NO: 1), an ionizable lipid (246-C10) : phospholipid (DOPE) : cholesterol : C16-PEG2000 ceramide (ceramide-PEG conjugate) was dissolved in ethanol at a molar ratio of 26.5 : 20 : 48.5 to 52.5 : 1.0 to 5.0 (cholesterol and lipid-PEG contents were adjusted so that the total sum of the molar ratios was 100), and lipid nanoparticles were prepared by mixing the organic and aqueous phases to achieve a weight ratio of mRNA : ionizable lipid of 1: 10. The size of the prepared nanoparticles was measured using dynamic light scattering (DLS) by a Malvern Zetasizer Nano (Malvern Instruments, UK) and the surface area was calculated as $4\pi r2$ (S in Table 9 below). Then, S was divided by the total amount of lipid (20 ug) used in the synthesis and then the relative proportion was expressed based on the value of LNPs comprising 1.0% PEG. (N in Table 9 below)

Then, after binding with 2 $\mu$g/ml of ApoE3 at RNA weight ratio: ApoE weight ratio=10:1, the amount of ApoE protein adsorbed on 0.2 $\mu$g/ml of LNPs was measured using the protocol of the ELISA kit (Abcam, USA), based on the concentration of mRNA contained in the nanoparticles after Triton-X treatment. Briefly, the ApoE protein included in the ELISA kit was prepared by serial dilutions of 2-fold starting from a concentration of 1 ug/ml, and the absorbance was measured to confirm the standard curve. ApoE absorbance measured at 450 nm using Infinite® 200 PRO NanoQuant (Tecan, USA) was substituted into the standard curve to confirm the concentration. The measured ApoE concentration was expressed as a relative ratio of the LNPs comprising 1.0% PEG. (n in Table 9 below) Then, to calculate the bound ApoE per nanoparticle, n was divided by N to calculate the ApoE adsorption rate accrording to PEG-lipid.

[Table 9]

| PEG (%) | Diameter (nm) | PDI | Particle surface area ($nm^2$) = S | Relative ratio of LNPs = N | Relative ApoE concentration | ApoE concentration/N |
|---|---|---|---|---|---|---|
| 1.0 | 104±10 | 0.057 | 135917.8363 | 1 | 1 | 1 |
| 1.5 | 60±10 | 0.148 | 45238.9248 | 3 | 3.3 | 1.1 |
| 3.0 | 42±8 | 0.154 | 22167.07315 | 6 | 2.8 | 0.47 |
| 5.0 | 40±5 | 0.128 | 20106.1888 | 7 | 4 | 0.57 |

As shown in Table 3 above, in the case of LNPs containing 3.0% or more of the lipid-PEG conjugate, ApoE adsorption was relatively reduced.

**Example 11-2. Measurement of intracellular nucleic acid delivery**

246-C10 LNPs comprising 1.5 to 5 mol% lipid-PEG conjugate and encapsulated with siFLuc were prepared as in the method of Example 2-2 above. Before transfection of LNPs into cells, HeLa-Luc cell line (MIT, USA) expressing luciferase was seeded at 0.01*10^6 cells/well and cultured in DMEM media (SH30022, Hyclone, USA) at 37°C, 0.5-3% $CO_2$. The siFLuc-encapsulated 246-C10 LNPs (comprising 1.5 to 5.0 mol% PEG-lipid) were treated with HeLa-Luc cells at a concentration of 10 nM (based on the siRNA concentration contained in the lipid nanoparticles) for one day. After treatment with 100 μl/well of Bright-Glo™ Luciferase Assay solution (promega, USA) for 10 min at room temperature, the lysed cells were measured for luminescence intensity using an Infinite M200 luminometer (Tecan, USA), and the results are shown in FIG. 10.

As shown in FIG. 10, intracellular nucleic acid delivery by the lipid nanoparticles decreased as the content of the lipid-PEG conjugate in the lipid nanoparticles increased.

**Example 12. Intracellular migration of lipid nanoparticles comprising mannose**

**Example 12-1. Diameter measurement of mannose-LNPs**

As in the method of Example 10-2 above, mannose LNPs comprising 1.5 to 3.0 mol% total lipid-PEG conjugate which is the sum of C16-PEG2000 ceramide and mannose-PEG-DSPE, and comprising 1.0 to 2.5 mol% mannose-PEG-DSPE conjugate were prepared, wherein the LNPs were encapsulated with mFluc (SEQ ID NO: 1) as shown in Table 10.

[Table 10]

| Name | total lipid-PEG conjugate | mannose-PEG-DSPE conjugate | ceramide-PEG conjugate |
|---|---|---|---|
| A1 | 1.5 | - | 1.5 |
| A2 | | 1.0 | 0.5 |
| B1 | 3 | - | 3.0 |
| B2 | | 2.5 | 0.5 |

**Example 12-2. Measurement of intracellular nucleic acid delivery of mannose LNPs**

The mannose-LNPs (A1 to B2) prepared in Example 12-1 were treated with HepG2 cells at a concentration of 100 ng/well based on the mRNA comprised in the lipid nanoparticles for 6 hours at 37°C and 0.5-3% $CO_2$. HepG2 cells are cells expressing CD206, a mannose-specific receptor. Thereafter, the cells were treated with 100 μl/well of Bright-Glo™ Luciferase Assay solution (promega, USA) and left at room temperature for 10 minutes, and the lysed cells were measured for luminescence intensity using an Infinite M200 luminometer (Tecan, USA), and the results are shown in FIG. 11.

As shown in FIG. 11, LNPs comprising mannose-PEG-DSPE exhibited superior intracellular nucleic acid delivery effect compared to LNPs without mannose-PEG-DSPE.

**Example 12-3. Confirmation of the effect of anti-CD206 antibody treatment**

Similar to the method of Example 12-2, mannose-LNPs comprising mFluc (SEQ ID NO: 1) were treated with HepG2 cells in the presence or absence of anti-CD206 antibody (Abcam, USA) 0.5 ug/well for 3 hours at 37°C, 0.5-3% CO2, and luminescence was measured, and the results are shown in FIG. 12a.

As a control, lipid nanoparticles comprising galactose-PEG-DSPE conjugate (hereinafter referred to as galactose-LNPs) instead of mannose-PEG-DSPE conjugate were prepared and treated to HepG2 cells in the presence or absence of anti-CD206 antibody in a similar method as described above, luminescence was measured, and the results are shown in FIG. 12b.

As shown in FIGs. 12a and 12b, the intracellular delivery effect of nucleic acids of mannose-LNPs compared to galactose-LNPs was significantly better than that of the control. In addition, the intracellular migration effect of mannose-LNPs according to one embodiment was reduced when treated with anti-CD206 antibody, but the intracellular nucleic acid delivery effect of galactose-LNPs was not significantly different with or without antibody treatment.

Thus, it was confirmed that the excellent intracellular nucleic acid delivery effect of mannose-LNPs according to one embodiment is due to the comprising mannose ligand.

## Example 13. Confirmation of LSEC targeting effect of mannose-LNPs

### Example 13-1. Ex vivo organ imaging

As in the method of Example 10-2, mannose-LNPs comprising a total of 3.0% lipid-PEG conjugate, 2.5% mannose-PEG-DSPE, and encapsulated with mCre were prepared and used to determine their biodistribution.

The mannose-LNPs (comprising 3.0 mol% total lipid-PEG (= 2.5 mol% mannose-PEG-DSPE and 0.5 mol% ceramide-PEG) were dialyzed in PBS for 16 hours to remove ethanol. LSL-tdTomato female and male mice at the age of 8 weeks (Jackson Laboratory, USA) were administered mannose-LNPs at a dose of 5 mg/kg, based on the mRNA (mCre) encapsulated in lipid nanoparticles, into the tail vein, and two days later, the mice were sacrificed and their organs were harvested, and tdTomato fluorescent protein expression was detected in each organ using IVIS equipment, and the results are shown in FIG. 13a.

As shown in FIG. 13a, it was confirmed that the mannose-LNPs exhibited high liver-specific luminescence intensity through ex vivo organ imaging, confirming that the mannose-LNPs according to one embodiment exhibit high biodistribution into the liver.

### Example 13-2. Liver histology image

Mannose-LNPs (comprising 3.0 mol% total lipid-PEG (= 2.5 mol% mannose-PEG-DSPE and 0.5 mol% ceramide-PEG) were prepared in the similar method as in Example 13-1. Then, the liver was fixed in 4% paraformaldehyde (PFA) (Sigma, USA). After paraffin embedding, the tissue was sectioned into 5 μm sections using a microtome. Then, they were incubated in pH 6.0 buffer (Dako, S1699) overnight at room temperature for heat-induced epitope retrieval (HIER). After HIER, tissue sections were stained with 4,6-diamidino-2-phenylindole (DAPI), α-SMA (Abcam, ab5694), Hnf4α (R&D, PP-H1415-00), and F4/80 (Abcam, Ab6640). Then, the fluorescence was detected by microscopy (EVOS® FL), and the results are shown in FIG. 13b.

As shown in FIG. 13b, the mannose-LNPs showed tomato fluorescence expression along the LSEC (blood vessel wall), confirming that the mannose-LNPs according to one embodiment effectively targeted LSECs.

### Example 13-3. FACS

Mannose-LNPs encapsulated with mCre (SEQ ID NO: 12) were prepared similarly to Example 13-1 above, and administered to LSL-tdTomato mice by tail vein, and 2 days later the mice were sacrificed, liver was harvested, and tomato fluorescence expression rate by hepatocytes (Hepatocyte, LSEC, Kupffer cell) was checked by FACS, and the results are shown in FIG. 13c. As a control, 246C10-LNPs comprising only 3 mol% C16 PEG2000 Ceramide as a lipid-PEG conjugate without mannose-PEG-DSPE were used.

Specifically, they were collected in 50 mL tube and digested with Liberase TM (Sigma-Aldrich) for 45 minutes at room temperature. After that, the cells were stained with specific antibodies and the results are shown in Figure 13c. The antibodies used to detect specific hepatocyte types were APC labeled anti-CD31 (biolegend, USA), FITC labeled anti-CD45 (biolegend, USA), PE/cy7 labeled anti-CD68 (biolegend, USA), and PE labeled anti-CD47 (biolegend, USA). Lastly, hepatocytes and non-parenchymal cells were analyzed by Novocyte 2060R (ACEA).

As shown in FIG 13c, the effect of targeting to Hepatocyte was 28%>10% for mannose-LNPs compared to control, and the tomato fluorescence expression rate decreased. The effect of targeting to LSEC was 25%<35% for mannose-LNPs compared to control, and the tomato fluorescence expression rate increased, and the effect of targeting to Kupffer cell was 4%<9%, which showed little change in tomato fluorescence expression.

### Example 13-4. siRNA delivery effect

Since FVII (Factor VII) is specifically expressed in hepatocytes, and FVIII (Factor VIII) is specifically expressed in LSECs (liver sinusoidal endothelial cells), the LSEC-targeting effect of mannose-LNPs was confirmed by knockout effect using siFVII and siFVIII.

Mannose-LNPs encapsulated with siRNA (siFVII or siFVIII) were prepared in a method similar to Example 10-2 above, and their diameters were measured, and the results are shown in Table 11 and Table 12, respectively.

[Table 11]

| siFVIII-LNPs | | |
|---|---|---|
| PEG (%) | Mean particle diameter(nm) | |
| | w/o mannose PEG | w/ mannose PEG |
| 1.5 | 65±6 | 128±8 |
| 3.0 | 42±8 | 101±10 |

[Table 12]

| siFVII-LNPs | | |
|---|---|---|
| PEG (%) | Mean particle diameter(nm) | |
| | w/o mannose PEG | w/ mannose PEG |
| 1.5 | 59±7 | 115±10 |
| 3.0 | 44±8 | 109±10 |

As shown in Table 11 and Table 12, the diameter of the nanoparticles increased in the case of mannose-LNPs.

The siRNA-encapsulated mannose-LNPs were intravenously injected into 57BL/6 female 7-week-old 20 g mice at siFVII 0.2mg/kg and siFVIII 0.5mg/kg concentrations based on the siRNA concentration contained in the lipid nanoparticles, and blood was collected via the tail vein 3 days after intravenous injection. Then, the blood was analyzed according to the protocol of the coaset FVII assay kit or the coaset FVIII assay kit, and a standard curve was drawn with the blood of mice treated with PBS to measure the amount of FVIII or FVII expression, and the results are shown in FIG. 14 and FIG. 15, respectively.

As shown in FIG. 14 and FIG. 15, the expression of hepatocyte-specific FVII was less effective in delivering nucleic acids than LNPs not comprising mannose-PEG-DSPE, but the expression of LSEC-specific FVIII was significantly inhibited in the case of mannose-LNPs. Thus, it was confirmed that mannose-LNPs according to examples have significantly superior LSEC-specific nucleic acid delivery efficiency.

## Example 16. Effect of mannose-LNP according to dose

In a similar method to Example 10-2, siFVIII-encapsulated mannose-LNPs were prepared to comprise only 3.0 mol% ceramide-PEG conjugate, or 0.5 mol% ceramide PEG conjugate and 2.5% mannose PEG-lipid conjugate (3.0% total PEG-conjugate). Then, the siRNA-loaded mannose-LNPs were intravenously injected into 57BL/6 female 7-week-old 20 g mice at a concentration of 0.5mg/kg, based on the siRNA concentration contained in the lipid nanoparticles, and blood was collected via the tail vein 2 days after intravenous injection. The blood was analyzed according to the protocol of the coaset FVIII assay kit, and FVIII expression was measured by plotting a standard curve with blood from mice treated with PBS, which is shown in FIG. 16.

As shown in FIG. 16, it was found that mannose-LNPs have superior LSEC-specific delivery effect than LNPs not comprising mannose-PEG-DSPE conjugate.

## Claims

1. A lipid nanoparticle comprising an ionizable lipid in which a 1,4-bis(3-aminopropyl) piperazine and an alkyl-epoxide are bonded; a phospholipid; cholesterol; and a mixture of lipid-PEG (polyethyleneglycol) conjugates,
   wherein the mixture of lipid-PEG conjugates comprises a mannose-PEG (polyethyleneglycol)-lipid conjugate.

2. The lipid nanoparticle according to claim 1, wherein the alkyl-epoxide is 1,2-epoxydodecane.

3. The lipid nanoparticle according to claim 1, wherein the phospholipid is one or more kinds selected from the group consisting of DOPE, DSPC, POPC, EPC, DOPC, DPPC, DOPG, DPPG, DSPE, Phosphatidylethanolamine, dipalmitoylphosphatidylethanolamine, 1,2-dioleoyl-sn-glycero-3-phosphoethanolamine, POPE, DOPS, and 1,2-dioleoyl-sn-glycero-3-[phospho-L-serine],

4. The lipid nanoparticle according to claim 1, wherein the lipid-PEG conjugate is one or more kinds selected from the group consisting of ceramide, dimyristoylglycerol (DMG), succinoyl-diacylglycerol (s-DAG), distearoylphosphatidylcholine (DSPC), distearoylphosphatidylethanolamine (DSPE), and cholesterol.

5. The lipid nanoparticle according to claim 1, wherein the mixture of lipid-PEG conjugates is comprised in 1.0 to 5.0 mol%.

6. The lipid nanoparticle according to claim 1, wherein the mannose-PEG-lipid conjugate is comprised in 0.5 to 4.5 mol%.

7. The lipid nanoparticle according to claim 1, wherein the lipid nanoparticle comprises 0.5 to 4.5 mol% of a lipid-PEG conjugate other than the mannose-PEG-lipid conjugate.

8. The lipid nanoparticle according to claim 1, wherein the lipid nanoparticle comprises the ionizable lipid : phospholipid : cholesterol : mixture of lipid-PEG conjugates at a molar ratio of 20 to 50 : 10 to 30 : 30 to 60 : 0.5 to 5.

9. The lipid nanoparticle according to claim 1, wherein the lipid nanoparticle has a pKa of 6.0 to 7.0.

10. The lipid nanoparticle according to claim 1, wherein the lipid nanoparticle specifically targets liver tissue.

11. The lipid nanoparticle according to claim 1, wherein the lipid nanoparticle specifically targets a LSEC (liver sinusoidal endothelial cell).

12. A composition for drug delivery, comprising (1) the lipid nanoparticle according to any one of claims 1 to 11; and (2) an anionic drug, a nucleic acid or a combination thereof.

13. The composition for drug delivery according to claim 12, wherein the anionic drug, nucleic acid or combination thereof is encapsulated inside of the lipid nanoparticle.

14. The composition for drug delivery according to claim 12, wherein the composition delivers a drug specifically to CD206 positive cells.

15. The composition for drug delivery according to claim 12, wherein the composition delivers the drug specifically to a LSEC (liver sinusoidal endothelial cell).

16. The composition for drug delivery according to claim 12, wherein the lipid nanoparticle has an average diameter of 70nm to 150nm.

17. The composition for drug delivery according to claim 12, wherein the anionic drug is one or more kinds selected from the group consisting of a peptide, a drug protein, a protein-nucleic acid structure, and an anionic biopolymer-drug conjugate.

18. The composition for drug delivery according to claim 12, wherein the nucleic acid is one or more kinds selected from the group consisting of small interfering ribonucleic acid (siRNA), ribosome ribonucleic acid (rRNA), ribonucleic acid (RNA), deoxyribonucleic acid (DNA), complementary deoxyribonucleic acid (cDNA), aptamer, messenger ribonucleic acid (mRNA), transfer ribonucleic acid (tRNA), antisense oligonucleotide, shRNA, miRNA, ribozyme, PNA and DNAzyme.

19. A pharmaceutical composition for preventing or treating liver disease, comprising (1) the lipid nanoparticle according to any one of claims 1 to 11; and (2) an anionic drug, a nucleic acid or a combination thereof.

20. The pharmaceutical composition according to claim 19, wherein the liver disease is one or more kinds selected from the group consisting of hepatitis B virus infection, acute liver failure, cirrhosis, liver fibrosis, liver fibrosis, hemophilia A, hemorrhagic necrosis, acute liver failure, and liver regeneration.

21. The pharmaceutical composition according to claim 19, wherein the lipid nanoparticle is specifically delivered to a LSEC (liver sinusoidal endothelial cell) in vivo.

【FIG. 1】

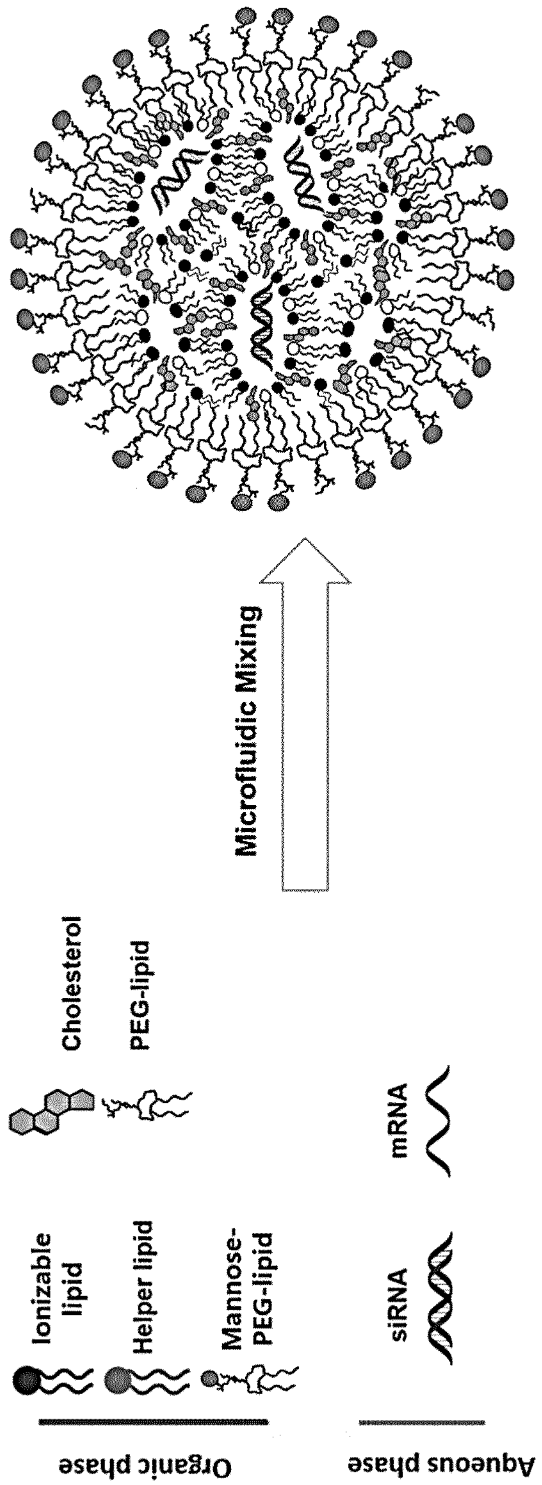
Organic phase
Ionizable lipid
Helper lipid
Mannose-PEG-lipid
Cholesterol
PEG-lipid
Aqueous phase
siRNA
mRNA
Microfluidic Mixing

【FIG. 2】

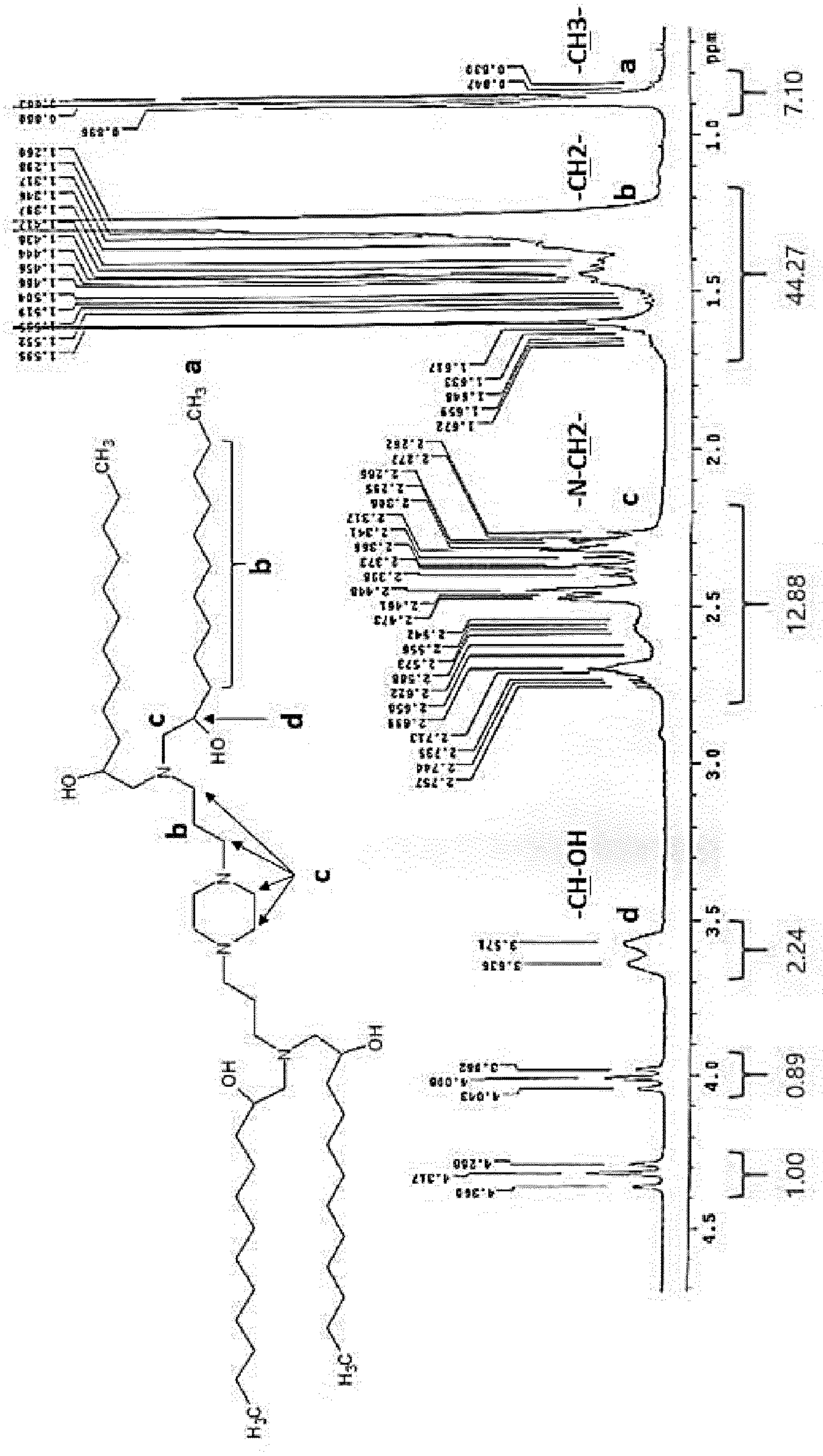


Room temperature 400 MHz 1H NMR spectrum of 246C10 in CDCl3.

【FIG. 3a】

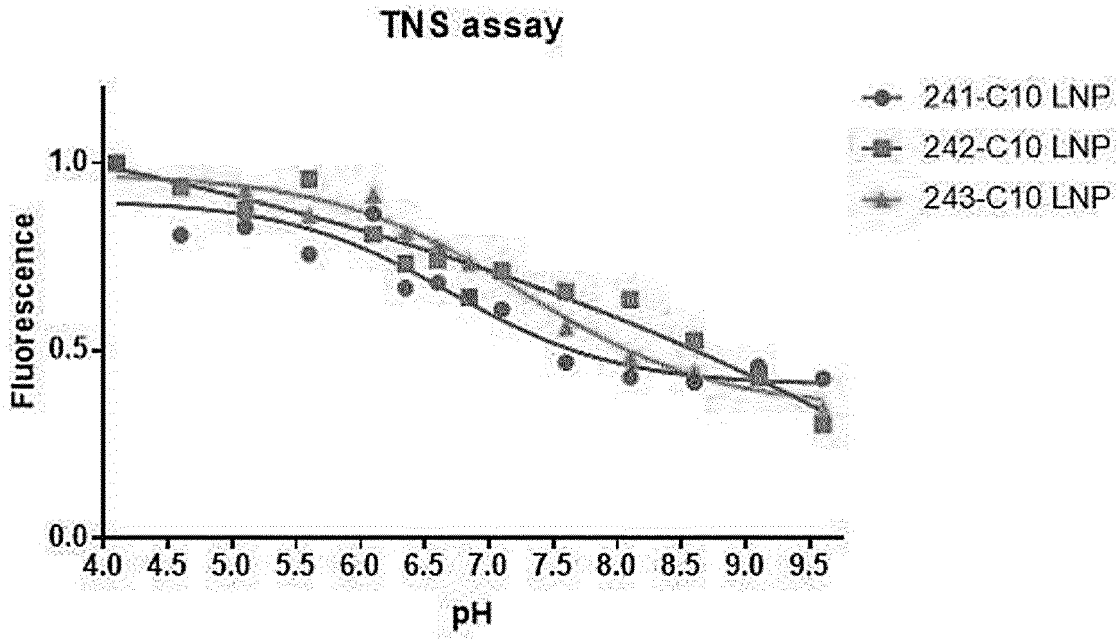
TNS assay
Fluorescence
pH
1.0
0.5
0.0
4.0
4.5
5.0
5.5
6.0
6.5
7.0
7.5
8.0
8.5
9.0
9.5
241-C10 LNP
242-C10 LNP
243-C10 LNP

【FIG. 3b】

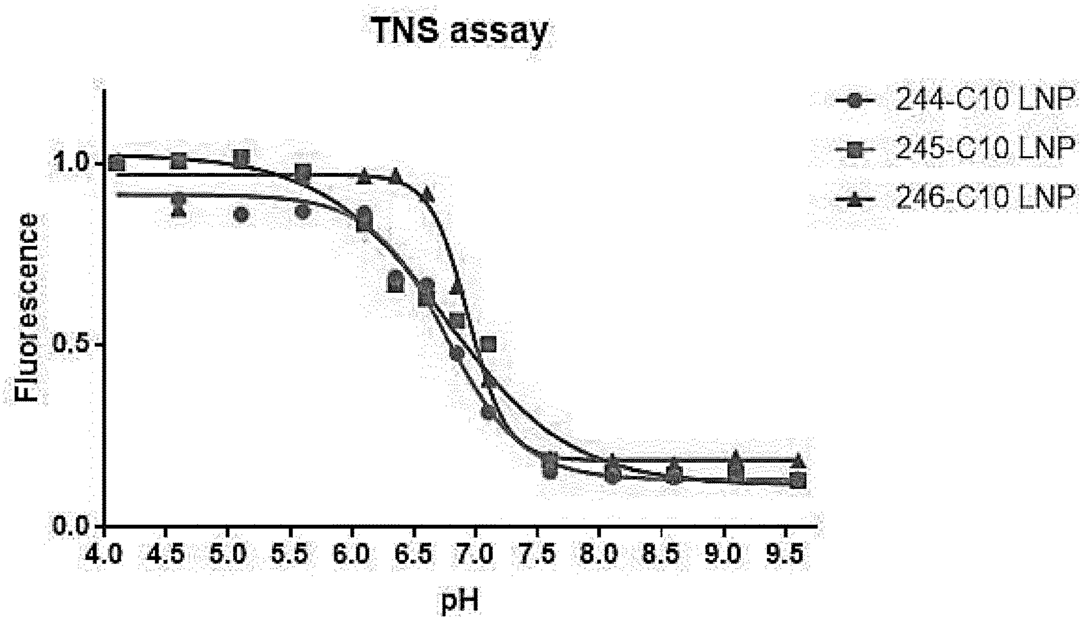
TNS assay
Fluorescence
1.0
0.5
0.0
4.0 4.5 5.0 5.5 6.0 6.5 7.0 7.5 8.0 8.5 9.0 9.5
pH
244-C10 LNP
245-C10 LNP
246-C10 LNP

【FIG. 4a】

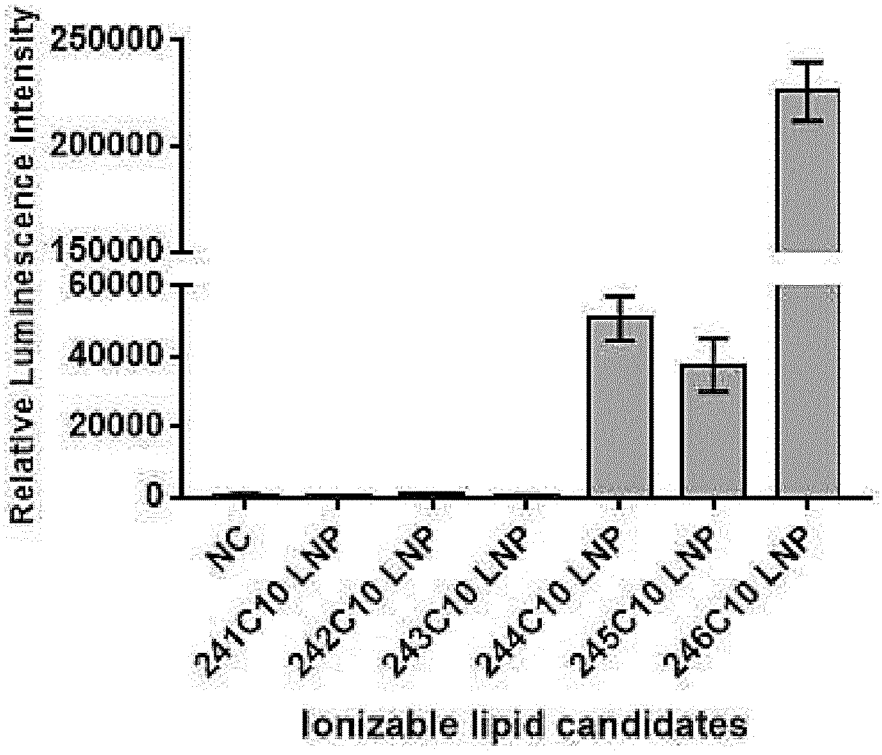
Relative Luminescence Intensity
250000
200000
150000
60000
40000
20000
0
NC
241C10 LNP
242C10 LNP
243C10 LNP
244C10 LNP
245C10 LNP
246C10 LNP
Ionizable lipid candidates

【FIG. 4b】

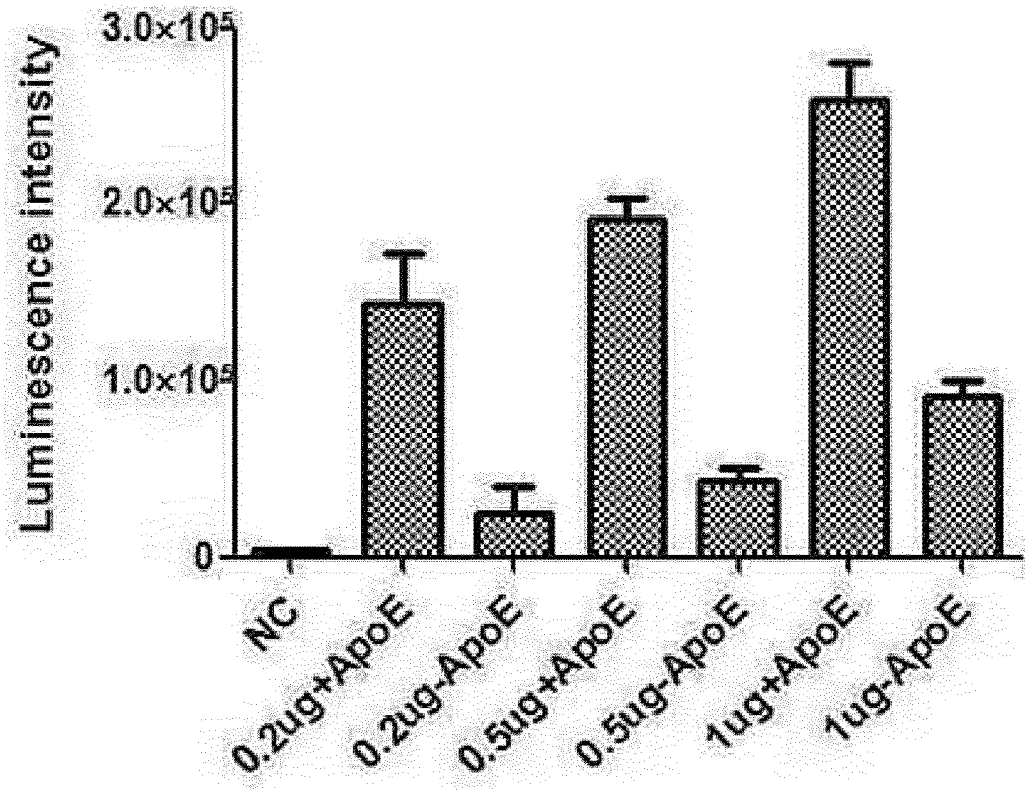
Luminescence intensity
3.0×10^5
2.0×10^5
1.0×10^5
0
NC
0.2ug+ApoE
0.2ug-ApoE
0.5ug+ApoE
0.5ug-ApoE
1ug+ApoE
1ug-ApoE

【FIG. 5a】

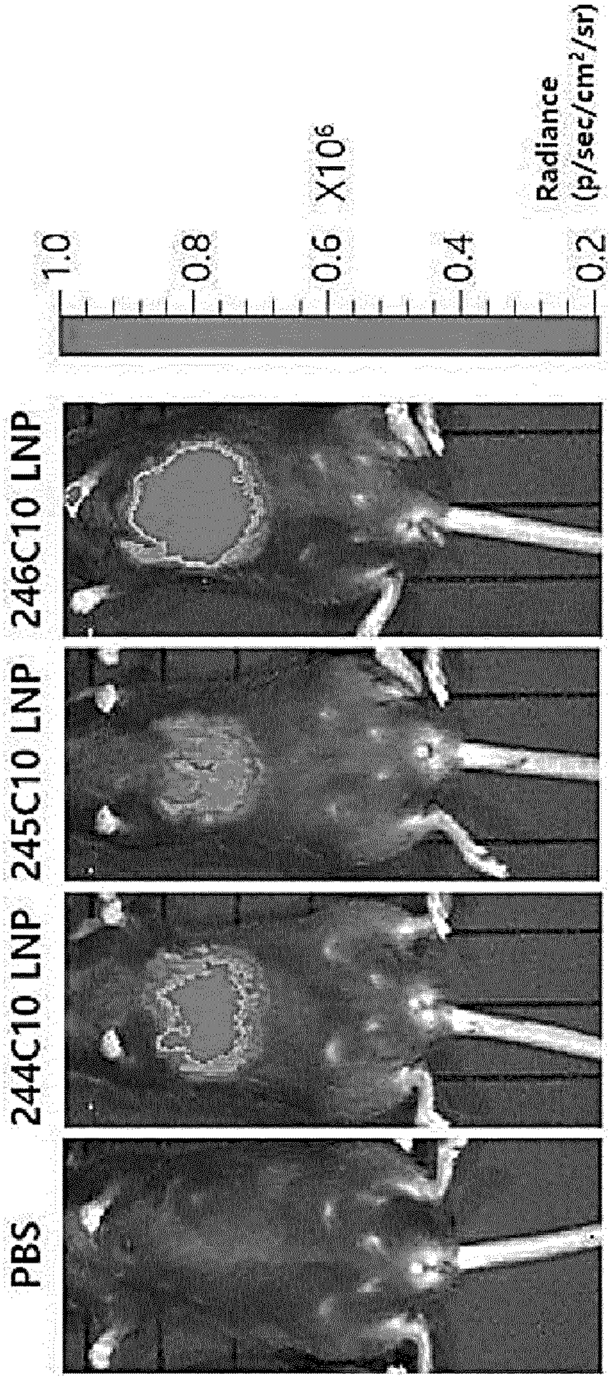
PBS
244C10 LNP
245C10 LNP
246C10 LNP
1.0
0.8
0.6
0.4
0.2
X10$^6$
Radiance
(p/sec/cm$^2$/sr)

【FIG. 5b】

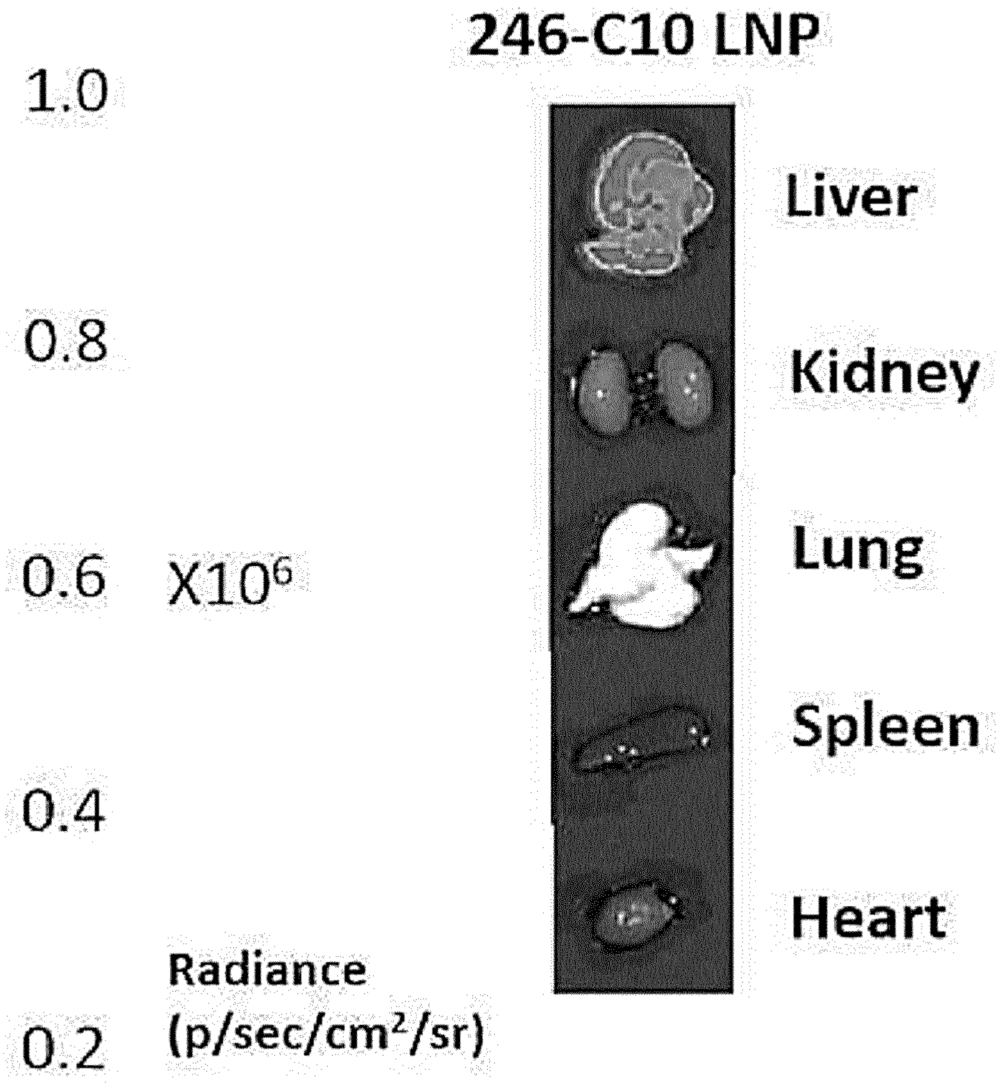
246-C10 LNP
1.0
0.8
0.6
X10^6
0.4
0.2
Radiance
(p/sec/cm²/sr)
Liver
Kidney
Lung
Spleen
Heart

【FIG. 6a】

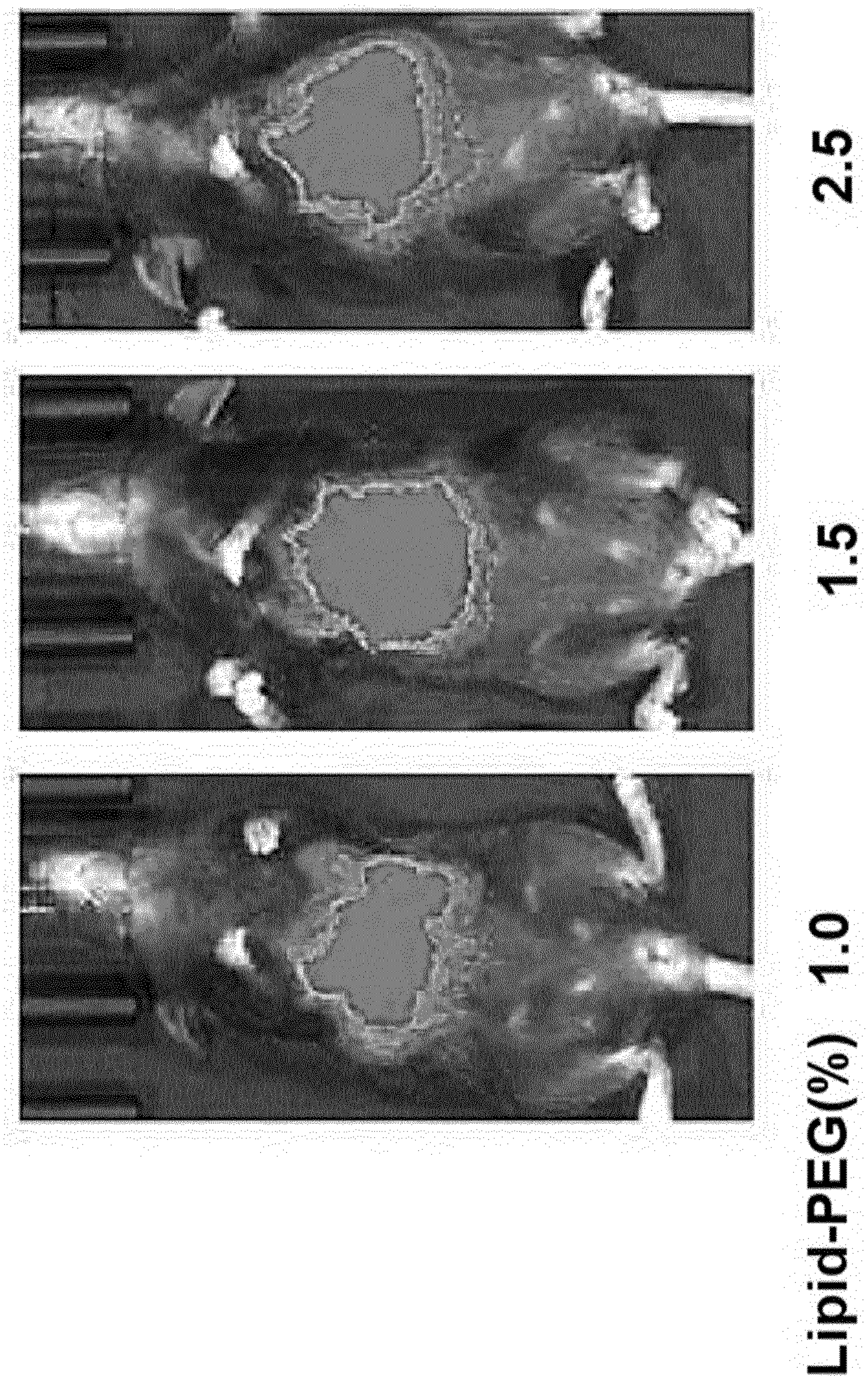

【FIG. 6b】

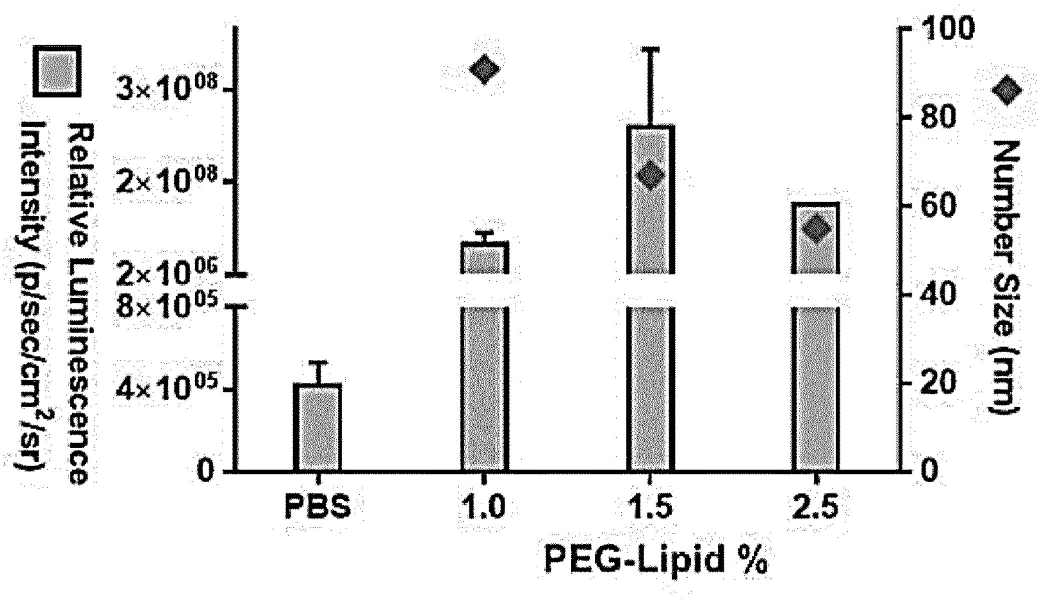
Relative Luminescence Intensity (p/sec/cm²/sr)
3×10⁰⁸
2×10⁰⁸
2×10⁰⁶
8×10⁰⁵
4×10⁰⁵
0
100
80
60
40
20
0
Number Size (nm)
PBS
1.0
1.5
2.5
PEG-Lipid %

【FIG. 7】

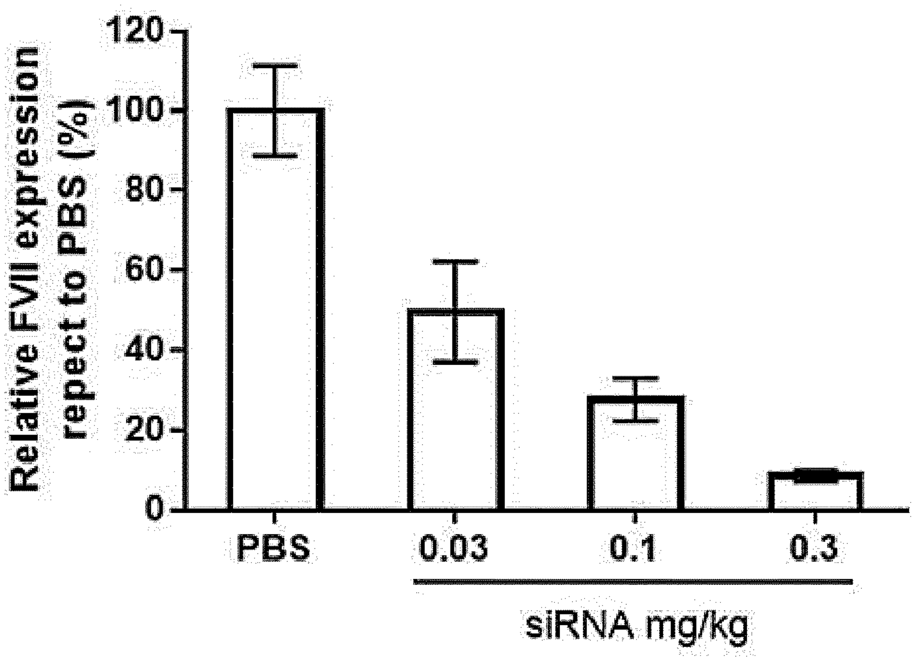
Relative FVII expression
repect to PBS (%)
120
100
80
60
40
20
0
PBS
0.03
0.1
0.3
siRNA mg/kg

【FIG. 8】

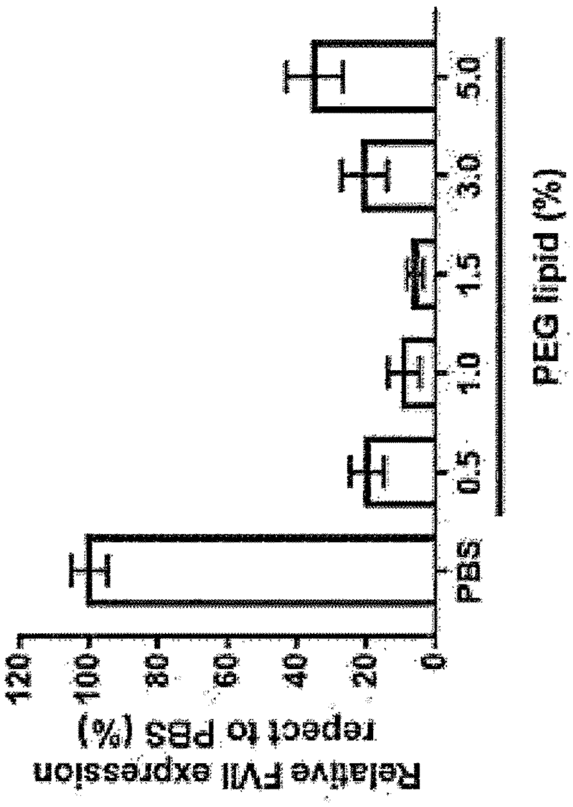


| Lipid - PEG (%) | Mean particle Size (nm) | PDI |
|---|---|---|
| 0.5 | 120 | 0.018 |
| 1.0 | 78 | 0.106 |
| 1.5 | 52 | 0.159 |
| 3.0 | 42 | 0.152 |
| 5.0 | 37 | 0.226 |

【FIG. 9】

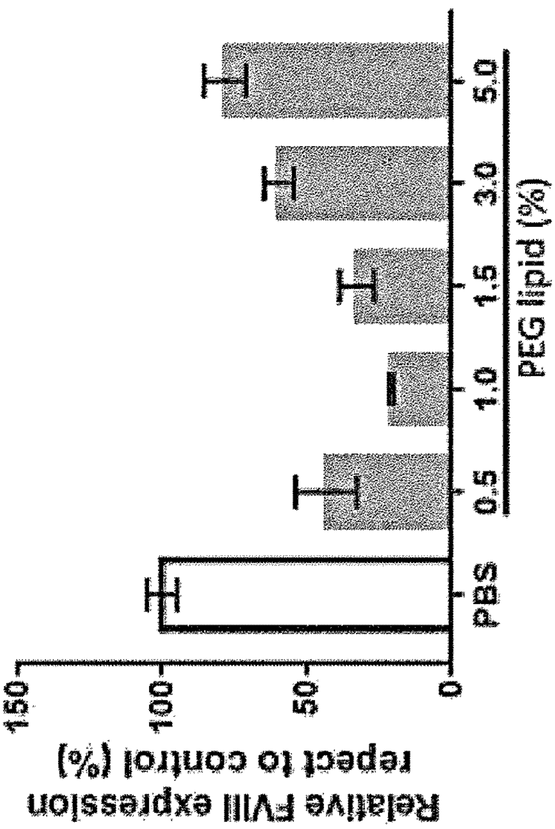


| Lipid - PEG (%) | Mean particle Size (nm) | PDI |
|---|---|---|
| 0.5 | 166 | 0.018 |
| 1.0 | 87 | 0.106 |
| 1.5 | 78 | 0.159 |
| 3.0 | 42 | 0.152 |
| 5.0 | 35.6 | 0.226 |

【FIG. 10】

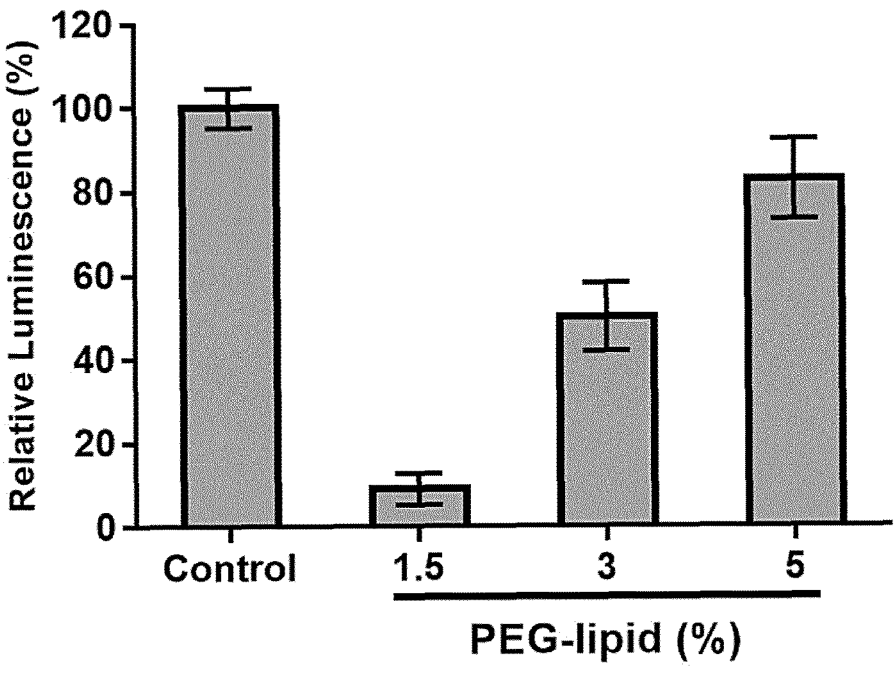
Relative Luminescence (%)
120
100
80
60
40
20
0
Control
1.5
3
5
PEG-lipid (%)

【FIG. 11】

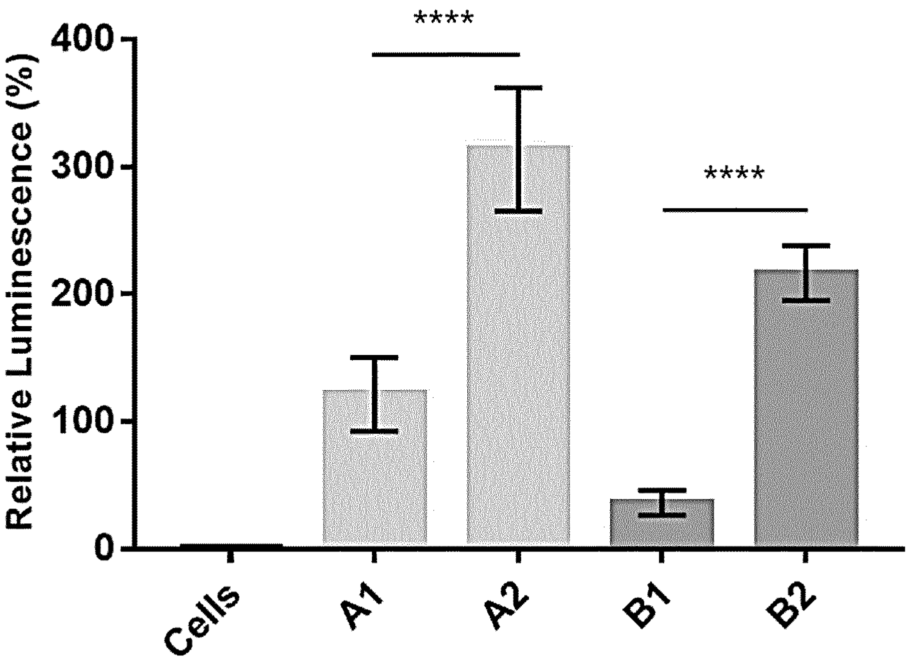

【FIG. 12a】

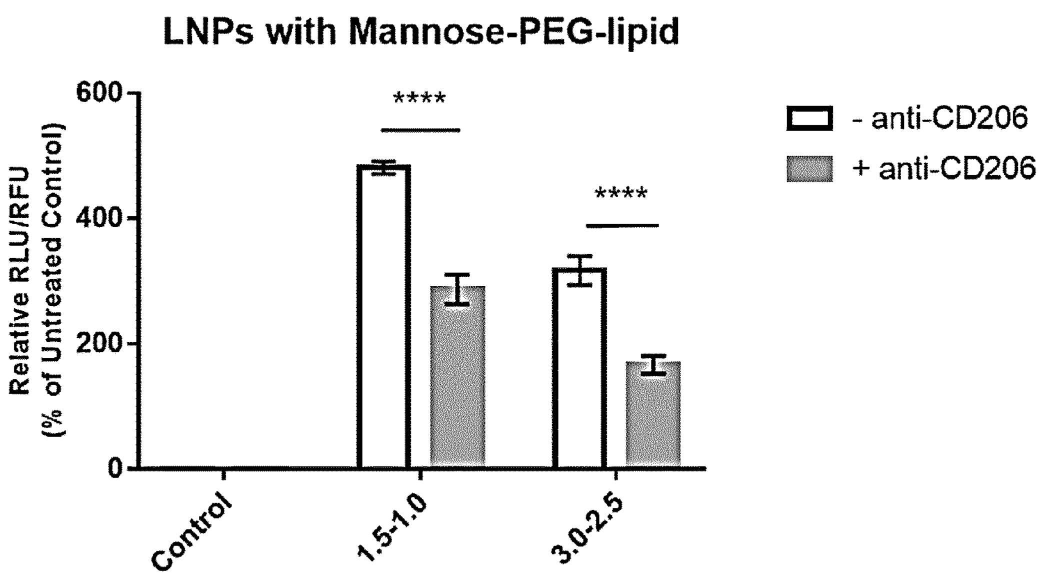
LNPs with Mannose-PEG-lipid
Relative RLU/RFU
(% of Untreated Control)
600
400
200
0
****
****
- anti-CD206
+ anti-CD206
Control
1.5-1.0
3.0-2.5

【FIG. 12b】

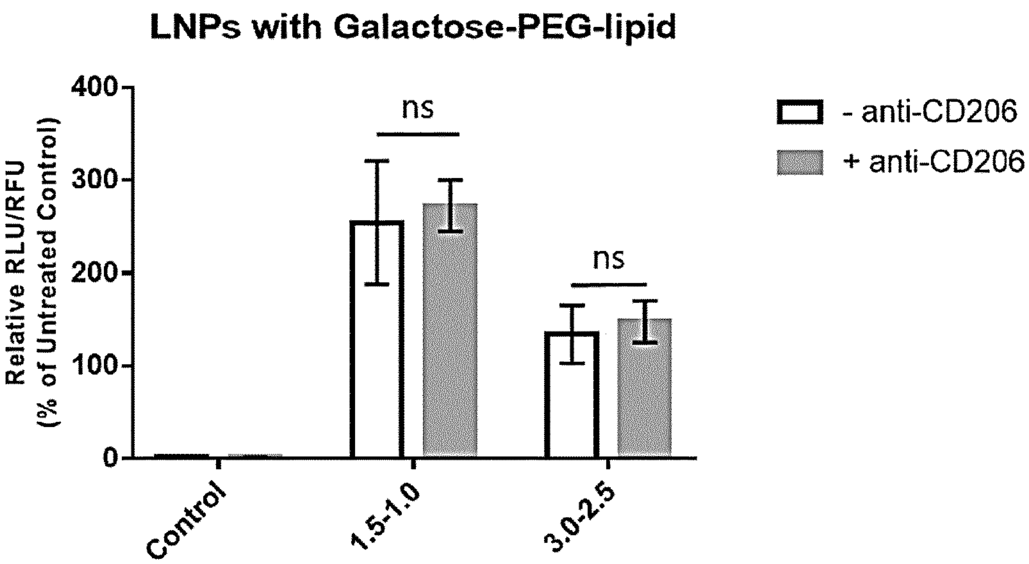
LNPs with Galactose-PEG-lipid
Relative RLU/RFU
(% of Untreated Control)
400
300
200
100
0
ns
ns
Control
1.5-1.0
3.0-2.5
- anti-CD206
+ anti-CD206

【FIG. 13a】

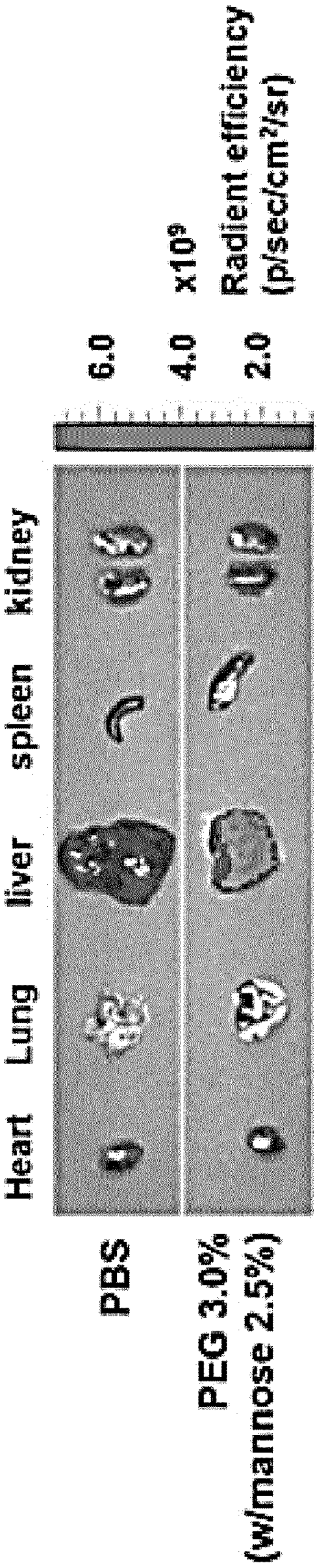
Heart
Lung
liver
spleen
kidney
PBS
PEG 3.0%
(w/mannose 2.5%)
6.0
4.0
2.0
x10$^9$
Radient efficiency
(p/sec/cm$^2$/sr)

【FIG. 13b】

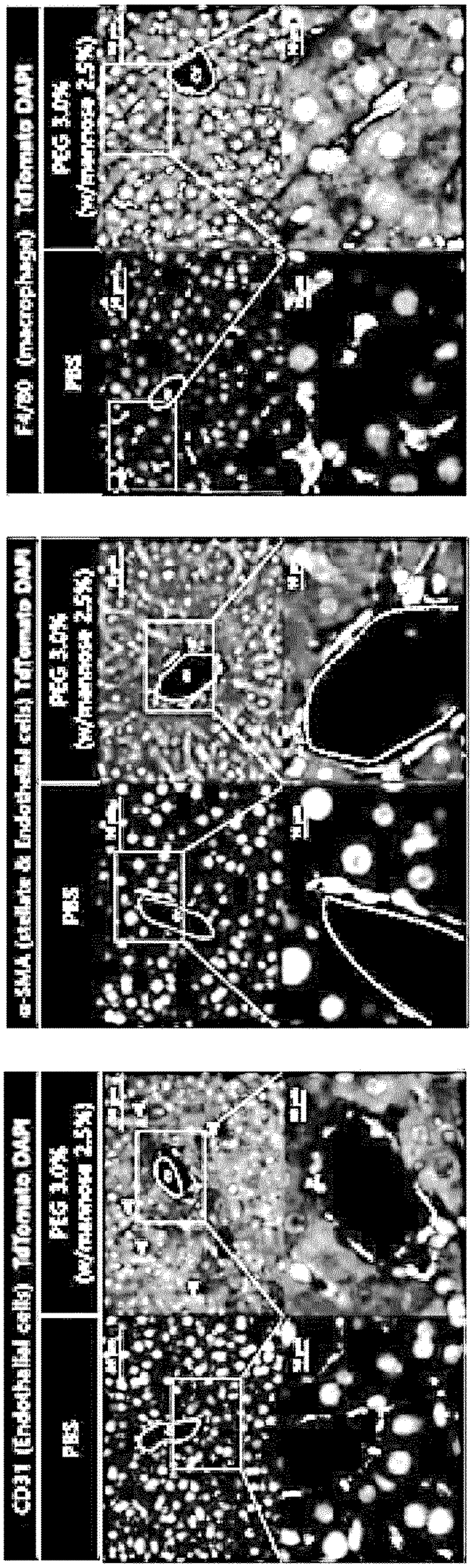
F4/80 (macrophage) TdTomato DAPI
PBS
PEG 3.0% (w/mannose 2.5%)
α-SMA (stellate & Endothelial cells) TdTomato DAPI
PBS
PEG 3.0% (w/mannose 2.5%)
CD31 (Endothelial cells) TdTomato DAPI
PBS
PEG 3.0% (w/mannose 2.5%)

【FIG. 13c】

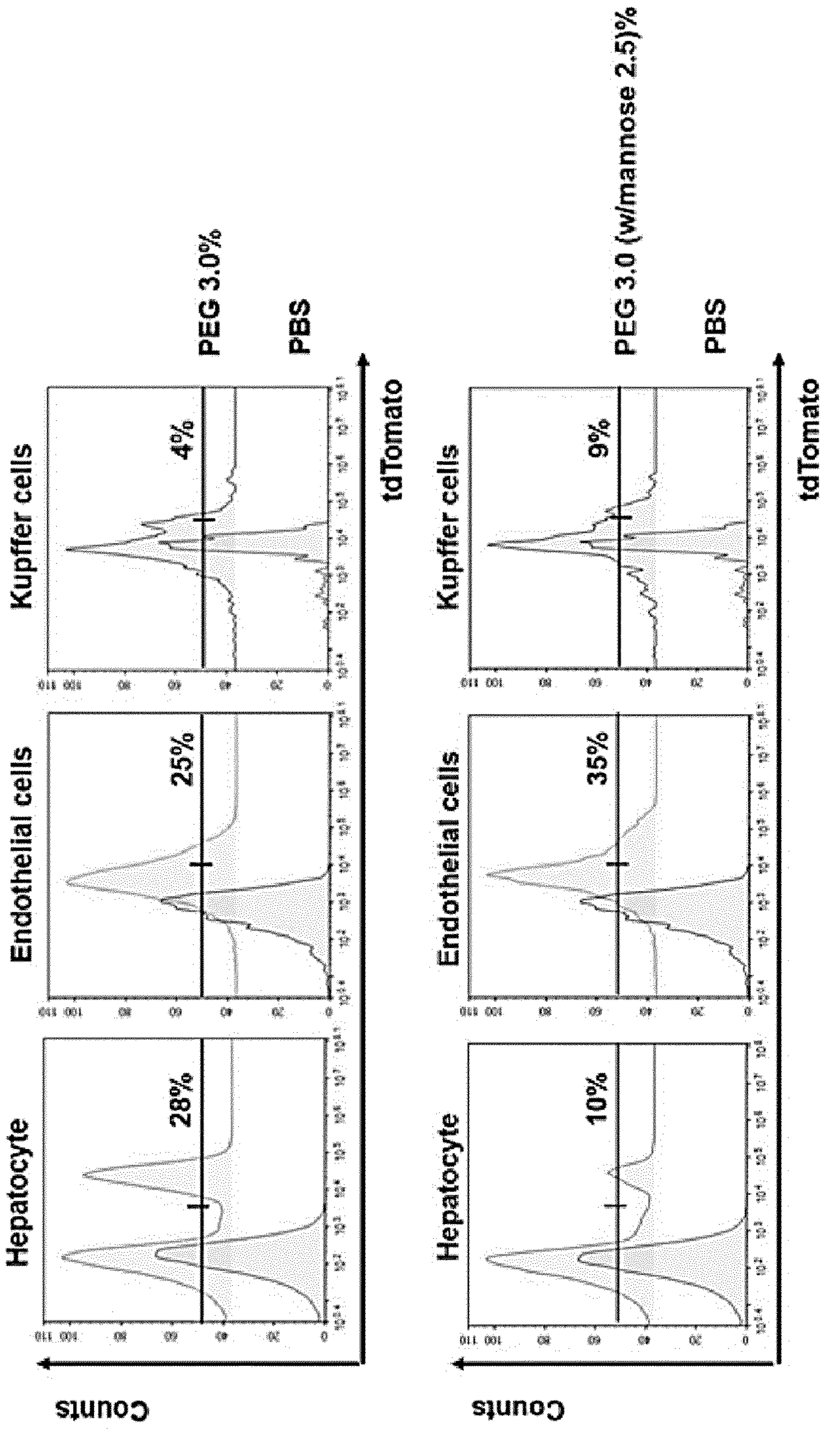
Hepatocyte
Endothelial cells
Kupffer cells
28%
25%
4%
PEG 3.0%
PBS
10%
35%
9%
PEG 3.0 (w/mannose 2.5)%
PBS
Counts
Counts
tdTomato
tdTomato

【FIG. 14】

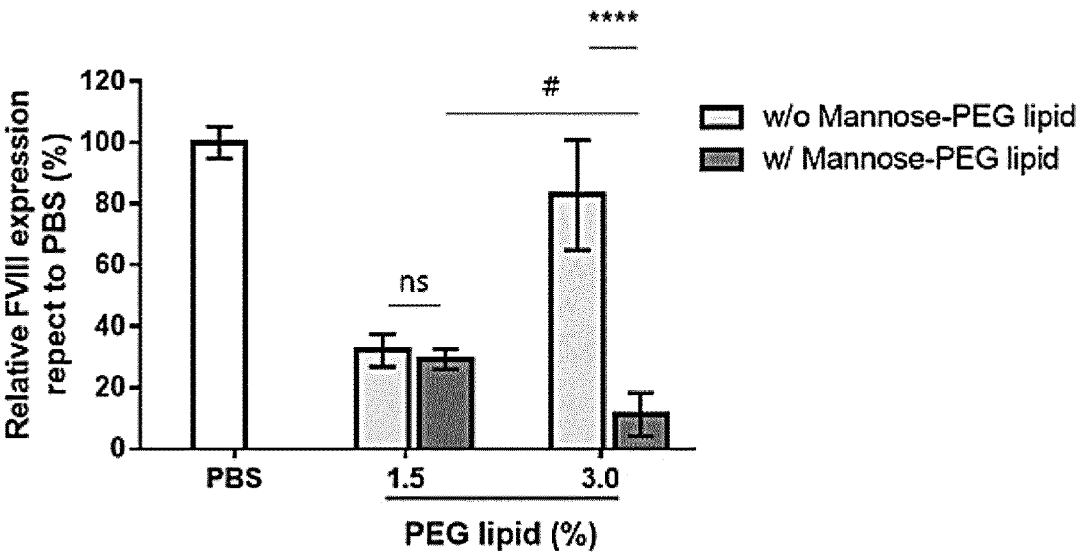

****
#
ns
120
100
80
60
40
20
0
Relative FVIII expression
repect to PBS (%)
PBS
1.5
3.0
PEG lipid (%)
w/o Mannose-PEG lipid
w/ Mannose-PEG lipid

【FIG. 15】

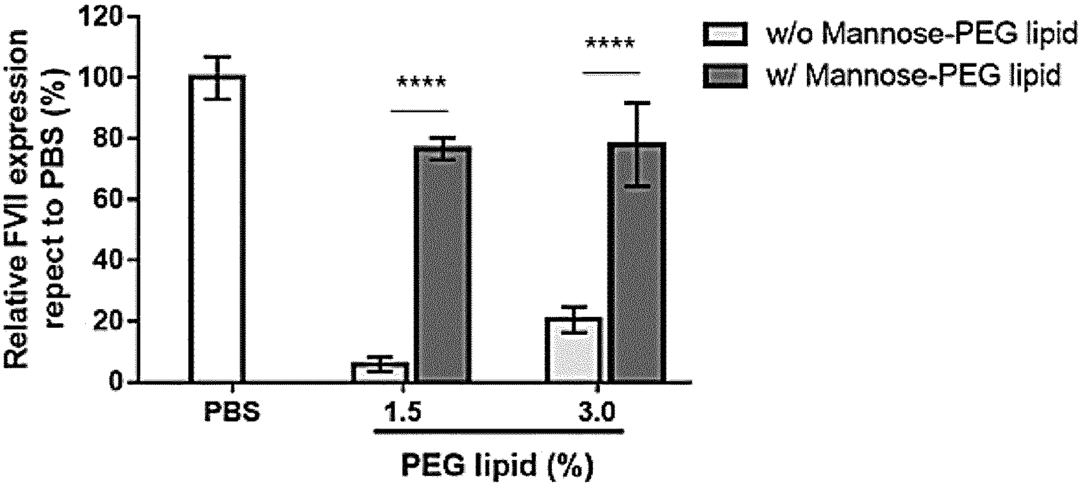
Relative FVII expression
repect to PBS (%)
120
100
80
60
40
20
0
****
****
PBS
1.5
3.0
PEG lipid (%)
w/o Mannose-PEG lipid
w/ Mannose-PEG lipid

【FIG. 16】

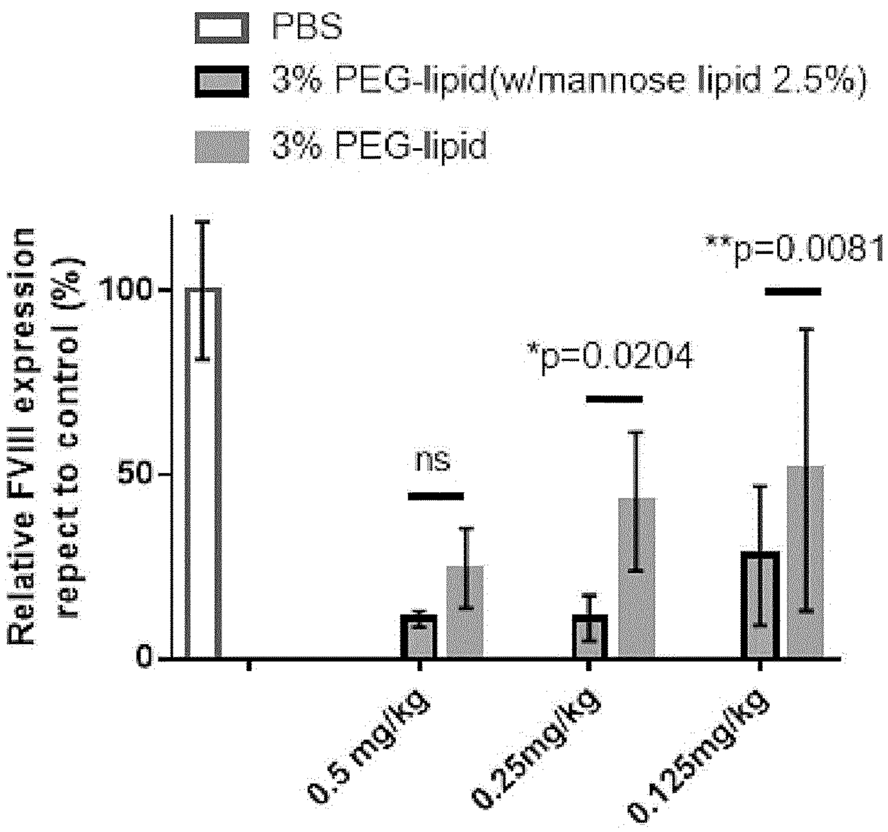
PBS
3% PEG-lipid(w/mannose lipid 2.5%)
3% PEG-lipid
Relative FVIII expression repect to control (%)
100
50
0
ns
*p=0.0204
**p=0.0081
0.5 mg/kg
0.25mg/kg
0.125mg/kg

**INTERNATIONAL SEARCH REPORT**

International application No.
**PCT/KR2021/019776**

**A. CLASSIFICATION OF SUBJECT MATTER**

**A61K 9/51**(2006.01)i; **A61K 48/00**(2006.01)i; **A61K 47/26**(2006.01)i; **A61K 47/10**(2006.01)i; **A61P 1/16**(2006.01)i; **A61P 31/20**(2006.01)i

According to International Patent Classification (IPC) or to both national classification and IPC

**B. FIELDS SEARCHED**

Minimum documentation searched (classification system followed by classification symbols)

A61K 9/51(2006.01); A61K 48/00(2006.01); A61K 9/127(2006.01); A61K 9/19(2006.01); C07K 14/525(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 1,4-비스(3-아미노프로필)피페라진(1,4-bis(3-aminopropyl)piperazine), PEG(polyethyleneglycol), 1,2-에폭시도데칸(1,2-epoxydodecane), 세라마이드(ceramide), LSEC(liver sinusoidal endothelial cell)

**C. DOCUMENTS CONSIDERED TO BE RELEVANT**

| Category* | Citation of document, with indication, where appropriate, of the relevant passages | Relevant to claim No. |
|---|---|---|
| Y | WO 2019-110067 A1 (AARHUS UNIVERSITET) 13 June 2019 (2019-06-13) See abstract; claims 1, 8 and 14; pages 6, 17, 19, 25 and 27; example 3; and figure 3A, A5, Ax and Ax-14. | 1-10,12-14,16-20 |
| A | | 11,15,21 |
| Y | ZHAO, Y. et al. Mannose-Modified Liposome Co-Delivery of Human Papillomavirus Type 16 E7 Peptide and CpG Oligodeoxynucleotide Adjuvant Enhances Antitumor Activity Against Established Large TC-1 Grafted Tumors in Mice. Int J Nanomedicine. 01 December 2020, vol. 15, pp. 9571-9586. See abstract; page 9581; and figure 1. | 1-10,12-14,16-20 |
| Y | KR 10-1685304 B1 (INDUSTRY-ACADEMIC COOPERATION FOUNDATION, YONSEI UNIVERSITY) 09 December 2016 (2016-12-09) See abstract; claim 7; paragraphs [0007], [0027] and [0060]-[0061]; and figure 1. | 2-3,10 |
| A | US 2020-0129445 A1 (MODERNATX, INC. et al.) 30 April 2020 (2020-04-30) See entire document. | 1-21 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 April 2022** | **01 April 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT

International application No.
**PCT/KR2021/019776**

C. DOCUMENTS CONSIDERED TO BE RELEVANT

| Category* | Citation of document, with indication, where appropriate, of the relevant passages | Relevant to claim No. |
|---|---|---|
| A | US 2019-0314291 A1 (MODERNATX, INC.) 17 October 2019 (2019-10-17) See entire document. | 1-21 |

Form PCT/ISA/210 (second sheet) (July 2019)

**INTERNATIONAL SEARCH REPORT**

International application No.
**PCT/KR2021/019776**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

**INTERNATIONAL SEARCH REPORT**
**Information on patent family members**

International application No.
**PCT/KR2021/019776**

| Patent document cited in search report | | | Publication date (day/month/year) | Patent family member(s) | | | Publication date (day/month/year) |
|---|---|---|---|---|---|---|---|
| WO | 2019-110067 | A1 | 13 June 2019 | None | | | |
| KR | 10-1685304 | B1 | 09 December 2016 | None | | | |
| US | 2020-0129445 | A1 | 30 April 2020 | AU | 2018-234828 | A1 | 19 September 2019 |
| | | | | CA | 3055653 | A1 | 20 September 2018 |
| | | | | EP | 3595727 | A1 | 22 January 2020 |
| | | | | JP | 2020-510072 | A | 02 April 2020 |
| | | | | WO | 2018-170336 | A1 | 20 September 2018 |
| US | 2019-0314291 | A1 | 17 October 2019 | AU | 2019-216307 | A1 | 09 July 2020 |
| | | | | CA | 3089117 | A1 | 08 August 2019 |
| | | | | EP | 3746052 | A1 | 09 December 2020 |
| | | | | JP | 2021-512090 | A | 13 May 2021 |
| | | | | WO | 2019-152557 | A1 | 08 August 2019 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **NOVINA ; SHARP.** *Nature,* 2004, vol. 430, 161-164
- **HIRKO et al.** *Curr. Med. Chem.,* 2003, vol. 10, 1185-93
- **MERDAN et al.** *Adv. Drug. Deliv. Rev.,* 2002, vol. 54, 715-58
- **SPAGNOU et al.** *Biochemistry,* 2004, vol. 43, 13348-13356
- **FILONAND ; PHILLIPS.** *Biochim. Biophys. Acta,* 1997, vol. 1329, 345-56
- **REN et al.** *Gene Therapy,* 2000, vol. 7, 764-768
- *CHEMICAL ABSTRACTS,* 7209-38-3
- **DE MESMAEKER et al.** *Curr Opin Struct Biol.,* 1995, vol. 5 (3), 343-55
- **NIELSEN PE ; EGHOLM M ; BERG RH ; BUCHARDT O.** Sequence-selective recognition of DNA by strand displacement with a thymine-substituted polyamide. *Science,* 1991, vol. 254, 1497-1500